# EUROPEAN PATENT APPLICATION

(11) **EP 4 678 736 A1**
(43) Date of publication of application: **14.01.2026**
(21) Application number: 24770745.8
(22) Date of filing: 08.03.2024
(51) Int. Cl.: C12N 5/00, C12M 1/00, C12M 1/26

(54) **CELL SOWING METHOD**

(30) Priority: 10.03.2023 JP 2023037583
(71) Applicant: Astellas Pharma, Inc., Chuo-ku Tokyo 103-8411 (JP)
(72) Inventor: SASAMATA, Miho, Tokyo 103-8411 (JP); SHIMOJO, Daisuke, Tokyo 103-8411 (JP)
(74) Representative: Hoffmann Eitle
(86) International application number: PCT/JP2024/008931
(87) International publication number: WO 2024/190639

(57) **Abstract**

The present invention relates to a method of seeding cells and a method of culturing cells. Specifically, the present invention relates to a method of seeding cells using a dispensing device, which comprises the steps of aspirating a cell suspension using the dispensing device, and dispensing the cell suspension to a single fixed point in a culture vessel using the dispensing device.

## Description

### Technical Field

The present invention relates to a method of seeding cells, and more specifically, to a method of seeding cells using a dispensing device. The present invention also relates to a method of culturing cells.

### Background Art

In recent years, the use of stem cells, such as human iPS cells, has enabled the generation of differentiated cells that were previously difficult to obtain. Furthermore, there have been cases where disease modeling was successfully performed *in vitro* using differentiated cells derived from iPS cells of affected patients, which has led to increased success rates in drug discovery research and the advancement of their use as cellular products (Sharma, A. et al., Cell Stem Cell. 2020; 26(3): 309-329).

The production of differentiated cells derived from human stem cells requires advanced techniques and long-term culturing. Thus, it is difficult to ensure consistent cell quality and reproducibility between experiments. For example, cell density is a critical factor for determining the differentiation bias, but is difficult to be controlled strictly. As a result, the properties of the final cells vary depending on the techniques of the individuals handling the experiment. In addition, even a highly skilled operator cannot perform the completely same experimental procedure repeatedly.

As described above, the cell density is a factor which influences the fate and the differentiation direction of the cell. As one traditionally known example, it has been reported that skeletal myoblasts, such as C2C12 cells, fuse and form muscle fibers when cultured at high density (Non Patent Literature 1). Further, in recent years, it has also been frequently reported that the quality of iPS cells, which are expected to be applied in drug discovery research and regenerative medicine, varies greatly depending on the cell density. It is reported, for example, that the expression level of transcription factors that serve as indicators of undifferentiation differs depending on the cell density (Non Patent Literature 2), and that the seeding density influences epigenetic memory, which in turn influences the differentiation direction (Non Patent Literature 3). In addition, it has also been reported that ununiformity of the cell density, such as a local bias in the cell density, in a culture vessel lead to differences in the resulting cell populations, that is, the differentiated cell populations. Specifically, it has been shown that, during induction of differentiation of iPS cells into hepatic cells, epithelial-like cells appear in a region with a low cell density, which results in reduced purity of the target cells (Non Patent Literature 4).

### Citation List

### Patent Literature

Patent Literature 1: JP Patent Publication (Kokai) No. 2021-023173

### Non Patent Literature

Non Patent Literature 1: Messina, G. et al., Molecular Biology of the Cell. 2005; 16: 1469-1480
Non Patent Literature 2: Myers, F.B. et al., Integr. Biol. 2013; 5: 1495-1506
Non Patent Literature 3: Kim, M.-H. et al., Stem Cell Research 2021; 56: 102534
Non Patent Literature 4: Graffmann, N. et al., PLoS ONE 2018; 13(7): e0200416

### Summary of Invention

### Technical Problem

In order to improve the cell quality, there have been reports of introducing a robot into drug discovery research and a cell product manufacturing process (Kanda, G.N. et al., eLife 2022; 11: e77007). Regarding cell culturing using such an automated cell culture apparatus, a method for improvement has been reported in which air bubbles are prevented from remaining inside wells during the seeding of a cell suspension in wells (Patent Literature 1). However, it remains difficult to produce homogeneous cells with high reproducibility, especially on a large scale. Furthermore, in order to obtain homogeneous cells with high reproducibility, it is desirable to develop a seeding method that minimizes a local bias in the cell density in the culture vessel.

Accordingly, an object of the present invention is to provide a method and means for uniformly seeding cells in a culture vessel. Another object of the present invention is to provide a method and means for culturing uniformly seeded cells to produce homogeneous cells with high reproducibility, especially on a large scale.

### Solution to Problem

The present inventors found that a method of dispensing cells during seeding of the cells from a dispensing device influences uniformity of the cells in a culture vessel. As a result of studying on conditions under which cells can be seeded uniformly, we found that the cell density in the culture vessel can be controlled without a local bias, which makes it possible to produce homogeneous cells on a large scale with high reproducibility, by dispensing a uniform cell suspension onto a single point of the culture vessel by using an automated system, and optionally leaving the cells to sit without shaking the culture vessel. We also found that homogeneous differentiated cells can be produced on a large scale with high reproducibility from the cells seeded in such a way.

For example, the present invention encompasses the following embodiments:
[1] A method of seeding cells using a dispensing device, the method comprising the steps of:
   aspirating a cell suspension using the dispensing device; and
   dispensing the cell suspension onto a single fixed point in a culture vessel using the dispensing device.
[2] The method according to [1], wherein the aspirating step and the dispensing step are each performed once or multiple times.
[2-1] The method according to [1], wherein the aspirating step and the dispensing step are each performed two to four times.
[3] The method according to [1] or [2], further comprising a step of leaving the culture vessel to sit without shaking after the dispensing step, thereby allowing the cells to adhere to the culture vessel.
[4] The method according to any one of [1] to [3], further comprising a step of stirring the cell suspension prior to the aspirating step.
[5] The method according to any one of [1] to [3], further comprising a step of stirring the cell suspension prior to the aspirating step, wherein the aspirating step and the dispensing step are each performed once or multiple times after the stirring step.
[6] The method according to [4] or [5], wherein after the stirring step, the aspirating step and the dispensing step are completed before the cells in the cell suspension sediment.
[7] The method according to any one of [1] to [6], wherein the single fixed point in the culture vessel is in a center portion of a bottom surface of the culture vessel.
[8] The method according to any one of [1] to [7], wherein the culture vessel is a flat-bottomed culture vessel.
[8-1] The method according to any one of [1] to [7], wherein the culture vessel is a quadrangular flat-bottomed culture vessel.
[9] The method according to any one of [1] to [8], wherein the cells are stem cells, preferably iPS cells.
[10] The method according to any one of [1] to [9], wherein the dispensing device is an automated dispensing device.
[11] A method of culturing cells in a culture vessel, the method comprising the steps of:
   seeding cells into the culture vessel by the method according to any one of [1] to [10];
      and
   culturing the cells in the culture vessel.
[11-1] The method according to [11], further comprising a step of transferring the culture vessel to an incubator after the cell seeding step.
[11-2] The method according to [11], further comprising a step of inducing differentiation of the cells.
[12] A method of dispensing a cell suspension using a dispensing device, the method comprising the steps of:
   aspirating the cell suspension using the dispensing device; and
   dispensing the cell suspension onto a single fixed point in a culture vessel using the dispensing device.
[13] A method of producing a cell stock, the method comprising the steps of:
   seeding cells into a culture vessel by the method according to any one of [1] to [10]; and
   culturing the cells in the culture vessel.
[13-1] The method according to [13], further comprising a step of transferring the culture vessel to an incubator.
[13-2] The method according to [13], further comprising a step of inducing differentiation of the cells.
[13-3] The method according to [13], further comprising a step of collecting the cultured cells from the culture vessel.
[14] A cell seeding device comprising:
   a dispensing device;
   a cell suspension holding part;
   a culture vessel holding part; and
   a control unit,
   wherein the control unit is configured to control such that:
      a cell suspension held at the cell suspension holding part is aspirated by the dispensing device; and
      the cell suspension aspirated by the dispensing device is dispensed onto a single fixed point in a culture vessel held at the culture vessel holding part.
[15] A cell culturing system comprising:
   the cell seeding device according to [14];
   an incubator;
   a transferring mechanism; and
   a control unit,
   wherein the control unit is configured to control such that:
      the culture vessel is transferred by the transferring mechanism from the culture vessel holding part of the cell seeding device to the incubator; and
      cells in the culture vessel are cultured in the incubator.
[16] A program for seeding cells using a dispensing device, the program being configured to cause the dispensing device to perform the steps of:
   aspirating a cell suspension held at a cell suspension holding part; and
   dispensing the cell suspension onto a single fixed point in a culture vessel held at a culture vessel holding part.
[16-1] The program according to [16], wherein the program causes the dispensing device to perform each of the aspirating step and the dispensing step once or multiple times (for example, two to four times).
[16-2] The program according to [16], wherein the program causes the dispensing device to further perform a step of stirring the cell suspension prior to the aspirating step.
[16-3] The program according to [16-2], wherein after the stirring step, the aspirating step and the dispensing step are completed before the cells in the cell suspension sediment.
[16-4] The program according to [16], wherein the single fixed point in the culture vessel is set within a center portion of a bottom surface of the culture vessel.

### Advantageous Effects of Invention

According to the method of seeding cells and the method of culturing cells, and the cell seeding device and the cell culturing system of the present invention, it is possible to uniformly seed cells into a culture vessel, which as a result enables production of homogeneous cells on a large scale with high reproducibility. Further, also when the cultured cells are induced to differentiate, it is possible to produce homogeneous differentiated cells on a large scale with high reproducibility. Therefore, the present invention is useful in fields such as cell culturing, drug discovery research, and the production of clinical cell preparations.

### Brief Description of Drawings

[Figure 1] Figure 1 shows (A) cell densities (the numbers of cells per field of view) and (B) a heat map based on the cell densities, when the cells are seeded under the condition (i) in Example 1.
[Figure 2] Figure 2 shows (A) cell densities (the numbers of cells per field of view) and (B) a heat map based on the cell densities, when the cells are seeded under the condition (ii) in Example 1.
[Figure 3] Figure 3 shows (A) cell densities (the numbers of cells per field of view) and (B) a heat map based on the cell densities, when the cells are seeded under the condition where shaking is performed ten times in Example 2.
[Figure 4] Figure 4 shows (A) cell densities (the numbers of cells per field of view) and (B) a heat map based on the cell densities, when the cells are seeded under the condition where shaking is performed 20 times in Example 2.
[Figure 5] Figure 5 shows (A) cell densities (the numbers of cells per field of view) and (B) a heat map based on the cell densities, when the cells are seeded under the condition where shaking is not performed in Example 2 and when cells are seeded at a dispensing speed of 890 µL/sec in Example 4.
[Figure 6] Figure 6 shows (A) cell densities (the numbers of cells per field of view) and (B) a heat map based on the cell densities, when the cells are seeded under the condition where shaking is performed each time in Example 3.
[Figure 7] Figure 7 shows (A) cell densities (the numbers of cells per field of view) and (B) a heat map based on the cell densities, when the cells are seeded at a dispensing speed of 179 µL/sec in Example 4.
[Figure 8] Figure 8 shows (A) cell densities (the numbers of cells per field of view) and (B) a heat map based on the cell densities, when the cells are seeded at a dispensing speed of 358 µL/sec in Example 4.
[Figure 9] Figure 9 shows (A) cell densities (the numbers of cells per field of view) and (B) a heat map based on the cell densities, when the cells are seeded at a dispensing speed of 1078 µL/sec in Example 4.
[Figure 10] Figure 10 shows (A) cell densities (the numbers of cells per field of view) and (B) a heat map based on the cell densities, when the cells are seeded at a dispensing speed of 1315 µL/sec in Example 4.
[Figure 11] Figure 11 shows (A) cell densities (the numbers of cells per field of view) and (B) a heat map based on the cell densities, when the cells are seeded at a dispensing speed of 1780 µL/sec in Example 4.
[Figure 12] Figure 12 shows (A) cell densities (the numbers of cells per field of view) and (B) a heat map based on the cell densities, when the cells are seeded at a dispensing speed of 2520 µL/sec in Example 4.
[Figure 13] Figure 13 shows (A) cell densities (the numbers of cells per field of view) and (B) a heat map based on the cell densities, when the cells are seeded at a dispensing speed of 4319 µL/sec in Example 4.
[Figure 14] Figure 14 shows (A) a graph of C.V. values and (B) a graph of Max/Mean values, when the cells are seeded at the respective dispensing speeds in Example 4.
[Figure 15] Figure 15 shows (A) cell densities (the numbers of cells per field of view) and (B) a heat map based on the cell densities, when the cells are seeded under the condition where a single point dispensing is performed and shaking is not performed in Example 5.
[Figure 16] Figure 16 shows (A) cell densities (the numbers of cells per field of view) and (B) a heat map based on the cell densities, when the cells are seeded under the condition where a double point dispensing is performed and shaking is not performed in Example 5.
[Figure 17] Figure 17 shows (A) cell densities (the numbers of cells per field of view) and (B) a heat map based on the cell densities, when the cells are seeded under the condition where a single point dispensing is performed and shaking is performed ten times in Example 5.
[Figure 18] Figure 18 shows (A) cell densities (the numbers of cells per field of view) and (B) a heat map based on the cell densities, when HEK293T cells are seeded in Example 6.
[Figure 19A] Figure 19A shows cell densities (cell nuclear areas per field of view) when the cells are seeded under the condition where a single point dispensing is performed in Example 7.
[Figure 19B] Figure 19B shows a heat map based on the cell densities (the cell nuclear areas per field of view) when the cells are seeded under the condition where the single point dispensing is performed in Example 7.
[Figure 20A] Figure 20A shows cell densities (cell nuclear areas per field of view) when the cells are seeded under the condition where a double point dispensing is performed in Example 7.
[Figure 20B] Figure 20B shows a heat map based on the cell densities (the cell nuclear areas per field of view) when the cells are seeded under the condition where the double point dispensing is performed in Example 7.
[Figure 21A] Figure 21A shows MyHC expression levels per cell ((skeletal muscle area) / (cell nuclear area) per field of view) after induction of differentiation, under the condition where the single point dispensing is performed in Example 7.
[Figure 21B] Figure 21B shows a heat map based on MyHC expression levels per cell ((skeletal muscle area) / (cell nuclear area) per field of view) after the induction of differentiation, under the condition where the single point dispensing is performed in Example 7.
[Figure 22A] Figure 22A shows MyHC expression levels per cell ((skeletal muscle area) / (cell nuclear area) per field of view) after induction of differentiation, under the condition where the double point dispensing is performed in Example 7.
[Figure 22B] Figure 22B shows MyHC expression levels per cell ((skeletal muscle area) / (cell nuclear area) per field of view) after the induction of differentiation, under the condition where the double point dispensing is performed in Example 7.
[Figure 23A] Figure 23A shows cell densities (the numbers of cells per field of view) when C2C 12 myoblasts are seeded under the condition where a single point dispensing is performed in Example 8.
[Figure 23B] Figure 23B shows a heat map based on the cell densities (the numbers of cells per field of view) when C2C12 myoblasts are seeded under the condition where the single point dispensing is performed in Example 8.
[Figure 24A] Figure 24A shows cell densities (the numbers of cells per field of view) when C2C 12 myoblasts are seeded under the condition where a double point dispensing is performed in Example 8.
[Figure 24B] Figure 24B shows a heat map based on the cell densities (the numbers of cells per field of view) when C2C 12 myoblasts are seeded under the condition where the double point dispensing is performed in Example 8.
[Figure 25A] Figure 25A shows MyHC expression levels per cell ((skeletal muscle area) / (cell nuclear area) per field of view) after induction of differentiation of C2C12 myoblasts, under the condition where the single point dispensing is performed in Example 8.
[Figure 25B] Figure 25B shows a heat map based on MyHC expression levels per cell ((skeletal muscle area) / (cell nuclear area) per field of view) after the induction of differentiation of C2C 12 myoblasts, under the condition where the single point dispensing is performed in Example 8.
[Figure 26A] Figure 26A shows MyHC expression levels per cell ((skeletal muscle area) / (cell nuclear area) per field of view) after induction of differentiation of C2C12 myoblasts, under the condition where the double point dispensing is performed in Example 8.
[Figure 26B] Figure 26B shows a heat map based on MyHC expression levels per cell ((skeletal muscle area) / (cell nuclear area) per field of view) after the induction of differentiation of C2C12 myoblasts, under the condition where the double point dispensing is performed in Example 8.

### Description of Embodiments

The present invention will be described in detail below.

The present invention relates to a method and a device for seeding cells, a method and a system for culturing cells, and the like. The present invention is partially based on the findings that, when seeding cells using a dispensing device, it is possible to improve the uniformity of the cell seeding into a culture vessel by performing procedures under conditions including dispensing a cell suspension onto a single fixed point in the culture vessel using the dispensing device, stirring the cell suspension before it is aspirated so that the cell suspension becomes homogeneous, not performing shaking after the cell suspension is dispensed, and dispensing at an appropriate speed, and that it is possible to produce homogeneous differentiated cells on a large scale with high reproducibility when the cells seeded in the above way are induced to differentiate.

As used herein, the term "cell seeding" refers to the act of spreading cells on a culture vessel or into a culture solution in the culture vessel, which is a procedure generally understood in the field of cell culturing. The term "culturing" of cells refers to at least one of maintaining cells, growing cells, differentiating cells, proliferating cells, or forming three-dimensional cellular aggregates, such as spheroids, in a culture vessel.

As used herein, the term "uniform" with respect to cells means that cells are not present with a local bias in the number of cells in a partial region within the culture vessel but are distributed evenly throughout. For example, uniformity can be determined by dividing the culture vessel into multiple regions and counting the number of cells per region, and then using any one or combination of the following: (1) C.V. value ((standard deviation (S.D.)) / mean); (2) Max/Mean value (a value calculated by dividing the maximum of the number of cells per region by the mean thereof); and (3) a heat map created based on the number of cells per region. As the C.V. value and the Max/Mean value become lower, the distribution can be determined to be more uniform. Further, as the visual differences in the shades of color on the heat map become smaller, the distribution can be determined to be more uniform and with less local bias in the cell density. As an example, the C.V. value may preferably be 13% or less and the Max/Mean value may preferably be 130% or less, but ultimately, the uniformity may be determined by visual observation of the heat map. On the other hand, as used herein, the term "homogeneous" with respect to cells means that cells have equal or nearly equal quality (such as proliferative capacity, differentiation potential, degree of differentiation, characteristics, and the like).

### (Method of Seeding Cells)

**In** one aspect, the present invention provides a method of seeding cells using a dispensing device, the method comprising the steps of:
aspirating a cell suspension using the dispensing device; and
dispensing the cell suspension onto a single fixed point in a culture vessel using the dispensing device.

The "cells" used herein are not particularly limited as long as they are intended to be seeded into a culture vessel for culturing, especially those for which uniform seeding into the culture vessel is desired. The cells may either be of a single type or a combination of multiple types. Examples of the cells may include stem cells, adipocytes, hepatic cells, renal cells, pancreatic cells, mammary gland cells, corneal cells, endothelial cells, epithelial cells, epidermal cells, smooth muscle cells, myoblasts, cardiac muscle cells, neural cells, glial cells, dendritic cells, chondrocytes, osteoblasts, osteoclasts, osteocytes, fibroblasts, blood cells, mesenchymal cells, and progenitor cells thereof. The cells may either be normal cells or diseased cells, such as tumor cells including cancer cells. **In** a preferred embodiment, the cells may be stem cells, such as induced pluripotent stem cells (iPS cells), embryonic stem cells (ES cells), and mesenchymal stem cells. The stem cells may either be undifferentiated (for example, undifferentiated iPS cells) or partially or completely differentiated cells derived from stem cells. The cells may either be naturally derived, or chemically or physically treated or genetically modified cells.

Further, the origin of the cells may not be particularly limited. For example, the cells may be derived from animals, preferably mammals. Specifically, the cells may be cells derived from primates (such as humans, monkeys, chimpanzees, and gorillas), laboratory animals (such as mice and rats), livestock (such as cattle, pigs, and rabbits), or companion animals (such as dogs and cats). For the purpose of preparing cell preparations, for example, cells derived from a human patient may be used as autologous cells. Further, for the purpose of preparing disease model cells, for example, cells isolated from a diseased site of a patient with that disease may be used, or known cell lines of the disease or cells in which a causative gene for the disease have been incorporated may be used.

The cells can be prepared by methods known in the field of the art, and for example, they can be cultured in an appropriate culture medium. The type of the culture medium may not be particularly limited, and for example, any basal culture medium for cell culturing, differentiation culture medium, or specialized culture medium for primary culture may be used. Specific examples of such culture media may include Dulbecco's Modified Eagle Medium (DMEM), StemFit medium, Glasgow's MEM (GMEM), RPMI 1640 medium, Ham's F12 medium, MCDB medium, and Williams' E medium. These examples may not be limited, and any culture medium containing components necessary for the proliferation or differentiation of cells can be used. Further, a culture medium supplemented with serum, various growth factors, or differentiation inducing factors may also be used.

As used herein, the term "dispensing device" refers to a device which includes a mechanism for aspirating and dispensing a cell suspension (for example, a pipette). The dispensing device may be operated either manually or automatically. Preferably, the dispensing device may be an automated dispensing device. In one embodiment, the automated dispensing device may be one that can precisely control the aspirating step and the dispensing step (for example, a dispensing device operated by LabDroid Maholo).

First, a cell suspension may be prepared. The term cell suspension refers to a liquid obtained by suspending cells in a liquid medium. The form of the cells in the cell suspension may either be as dispersed single cells or as a cell cluster composed of multiple cells. The liquid medium may vary depending on the type of the cells used, and can be selected to be suitable for the subsequent cell culturing. Examples of the liquid medium may include Dulbecco's Modified Eagle Medium (DMEM), StemFit medium, Glasgow's MEM (GMEM), RPMI 1640 medium, Ham's F12 medium, MCDB medium, and Williams' E medium.

The concentration of the cell suspension can be suitably set by a person skilled in the art by taking into consideration factors such as the type of cells, the volume of the liquid to be dispensed, the presence or absence of previously added culture solution in the culture vessel, and the cell density after seeding the cell suspension into the culture vessel. The cell density after the seeding of the cell suspension may vary depending on the type of the cells and the purpose of culturing, and may be, for example, 10² to 10⁶ cells/cm², 10² to 10⁵ cells/cm², or 10³ to 10⁶ cells/cm², preferably 10³ to 10⁵ cells/cm². For example, the cell density in the culture vessel may be set to be higher for a case where differentiation is induced after the culturing than for a case of simple cell culturing. It may be also assumed that the cell density is set to be lower for the case with the differentiation induction.

According to the present method of seeding cells, the cell suspension may be aspirated using a dispensing device. The volume of the cell suspension to be aspirated can be determined depending on the total volume of the cell suspension to be dispensed into the culture vessel and the number of dispensing operations, and may be limited by the maximum aspirating volume of the dispensing device. For example, if a total volume of 5 to 15 mL of the cell suspension is dispensed over one to four times, 1.25 to 15 mL of the cell suspension may be aspirated per operation. When the aspirating step is performed multiple times, the volume of the cell suspension to be aspirated may either be the same or different between operations. The aspirating speed may not be particularly limited as long as it is a speed within the commonly practiced range.

Subsequently, the cell suspension may be dispensed onto a single fixed point in the culture vessel using the dispensing device. As used herein, the term "culture vessel" refers to a container into which cells are seeded by the present method of seeding cells and then the seeded cells are cultured. Culture vessels are well known in the field of the art and are commercially available in various forms and shapes. A person skilled in the art can suitably select the shape, material, and size (culture surface area) of the culture vessel depending on the type of cells used, the form and purpose of the culturing, and the scale of the culturing. Examples of the culture vessels may include wells of a plate for culturing, such as single or multi-well culture plates, specifically wells of a 1-well plate, each well of a 6-well plate, each well of a 12-well plate - Petri dishes, and dishes. In a case where a single plate has multiple wells, like a multi-well plate, each well is considered as a culture vessel herein. In one embodiment, the culture vessel may be a well of a 1-well plate, a well in a 6-well plate, or a well in a 12-well plate. Further, the shape of the culture vessel may not be limited, and may be round, oval, or quadrangle (such as square or rectangle), and the bottom may be flat or round. When the uniform cell density is desired, such as in a case of stem cells, it may be preferable to use a flat-bottomed culture vessel. In one embodiment, the culture vessel may be a well of a 1-well plate with a quadrangular flat bottom. The material of the culture vessel may also not be particularly limited as long as it is generally used in cell culturing. Examples of the material may include, but are not limited to: quartz; inorganic glass, such as glass and borosilicate glass; carbon; metals, such as gold, silicon, nickel, titanium, and aluminum; polyolefins, such as polyethylene and polypropylene; polyesters, such as polybutylene terephthalate (PBT) and polyethylene terephthalate (PET); cyclic olefin resins; acrylic resins, such as polymethyl methacrylate (PMMA); and epoxy resins. The size (culture surface area) of the culture vessel may also not be particularly limited. For example, it may be suitably set as 3 cm² or more, such as 9 cm² or more, 90 cm² or more, 3 to 500 cm², 3 to 200 cm², 3 to 100 cm², 9 to 500 cm², 9 to 200 cm², 9 to 100 cm², 20 to 500 cm², 20 to 200 cm², 20 to 100 cm², 40 to 500 cm², 40 to 200 cm², 40 to 100 cm², 60 to 500 cm², 60 to 200 cm², 60 to 150 cm², 60 to 140 cm², 60 to 120 cm², 60 to 100 cm², 70 to 500 cm², 70 to 200 cm², 70 to 150 cm², 70 to 140 cm², 70 to 120 cm², 70 to 100 cm², 80 to 500 cm², 80 to 200 cm², 80 to 150 cm², 80 to 140 cm², 80 to 100 cm², 90 to 500 cm², 90 to 200 cm², 90 to 150 cm², 90 to 140 cm², 90 to 100 cm², or 95 cm², for example. In one embodiment, the culture surface area of the culture vessel may be 3 to 100 cm². In one embodiment, the culture surface area of the culture vessel may be 60 to 150 cm² (for example, 60 to 120 cm², 60 to 100 cm², 70 to 150 cm², 70 to 120 cm², or 70 to 100 cm²). In one embodiment, the culture surface area of the culture vessel may be 90 to 100 cm². In one embodiment, the culture vessel may be a well of a 1-well plate or a well of a multi-well plate having a culture surface area of 3 to 100 cm². In one embodiment, the culture vessel may be a well of a 1-well plate having a culture surface area of 60 to 150 cm² (for example, 60 to 120 cm², 60 to 100 cm², 70 to 150 cm², 70 to 120 cm², or 70 to 100 cm²). In one embodiment, the culture vessel may be a well of a 1-well plate having a culture surface area of 90 to 100 cm².

In one embodiment, a culture solution may be previously added to the culture vessel. For example, by previously adding the culture solution so as to achieve the cell density suitable for cell culturing (the cell density in the culture vessel) based on the concentration and the dispensing volume of the cell suspension, it is possible to adjust the final cell density. The culture solution may vary depending on the type of cells used and the purpose of the cell culturing, and can be suitably selected by a person skilled in the art. The culture solution may either be the same as or different from that used for the cell suspension.

In one embodiment, the culture vessel may be partially or entirely coated with a coating agent (for example, a culture substrate). Alternatively, the culture solution previously added to the culture vessel may contain the coating agent. The coating agent may not be particularly limited as long as it is commonly used in cell culturing. For example, examples of a coating agent for iPS cells may include iMatrix-511 (manufactured by Nippi), Matrigel (registered trademark), and vitronectin. In another embodiment, feeder cells may be cultured in the culture vessel. Such procedures commonly performed in cell culturing may also be suitably performed in the present invention.

The phrase "dispensing onto a single fixed point" means that the cell culture solution is dispensed onto a single point in the culture vessel, and that, even if the dispensing step is repeated multiple times, the cell suspension is dispensed onto the completely or substantially the same point. This single point may preferably be located on the bottom surface of the culture vessel, particularly at the center portion of the bottom surface of the culture vessel. The term "center portion" refers to the center or approximate center.

The volume of the cell suspension to be dispensed and the number of dispensing operations may be determined based on the total volume of the cell suspension to be dispensed into the culture vessel, and may be approximately equal to the volume and the number of times, respectively, of the aspiration of the cell suspension, as described above. If the dispensing volume per operation is too small or the number of dispensing operations is too large, it is considered that cells are concentrated at the center of the culture vessel. Therefore, the dispensing volume and the number of dispensing operations may be determined by taking into consideration factors such as the type of the cells, the concentration of the cell suspension, and the type of the liquid medium.

The dispensing speed of the cell suspension may be a factor that influences the uniform seeding of the cells into the culture vessel. An appropriate speed can be selected by taking into consideration factors such as the cell concentration and viscosity of the cell suspension, the volume of the cell suspension to be dispensed, and the height from which the cell suspension is to be dispensed. In one embodiment, the applicable dispensing speed may be 350 to 1350 µL of cell suspension per second, preferably 850 to 1100 µL of cell suspension per second. For example, a dispensing speed suitable for the cell suspension to be used can be determined by conducting experiments as described in Example 4. As an example, when a cell suspension prepared by suspending the cells in a culture medium at a concentration of 10² to 10⁷ cells/mL, preferably 10⁴ to 10⁶ cells/mL is used and is dispensed three times so that the volume per operation is approximately 5 mL, the dispensing speed can be set to 350 to 1350 µL/sec, preferably 850 to 1100 µL/sec. Although iPS cells were used in Example 4 for considering conditions, the type of the cells may not be particularly limited, and a similar dispensing speed can be applied. The dispensing speed may either be constant or variable within a certain range (for example, the dispensing speed may vary during a single dispensing operation, or different dispensing operations may be performed at different dispensing speeds).

The height from which the cell suspension is to be dispensed (that is, the distance from the bottom surface of the culture vessel to the dispensing point at the tip end of the dispensing device) may be such that the tip end of the dispensing device does not collide with the bottom surface of the culture vessel, and may preferably be 10 mm or more above the bottom surface of the culture vessel in order to avoid ununiformity of the cell density due to air bubbles generated during the dispensing. A height that prevents splashing of the cell suspension or the culture solution during the dispensing (for example, approximately 5 cm) may be permissible. In one embodiment, the dispensing height may be such that the dispensing device is not inserted into the culture vessel, and may be, for example, at or above the height of the wall of the culture vessel. In another embodiment, the dispensing height may be 10 mm or more above the bottom surface of the culture vessel, and the dispensing may be performed in a state in which the dispensing device is inserted inside the culture vessel.

In one embodiment, the cell suspension may not be dispensed in the form of sprayed droplets.

In the present method of seeding cells, the aspirating step and the dispensing step may each be performed once or multiple times, for example, two to four times. The number of times may be set depending on the total volume of the cell suspension to be dispensed and the dispensing volume per operation.

**In** one embodiment, the present method of seeding cells may further comprise a step of leaving the culture vessel to sit without shaking after the final dispensing step, thereby allowing the cells to adhere to the culture vessel. The sitting time can be suitably set depending on the type of the cells and the treatment of the culture medium, and may be, for example, 1 to 30 minutes, preferably 5 to 20 minutes.

As used herein, the "shaking" refers to the act of swinging the culture vessel in an approximately horizontal direction. Shaking methods commonly performed in the field of cell culturing may include reciprocal shaking, rotary shaking, and figure-eight shaking. A person skilled in the art can suitably select the type of shaking depending on the purpose.

According to the present method of seeding cells, it may be preferable not to shake the culture vessel after the dispensing step. However, some cases may be assumed in which shaking may be performed, such as a case where the cell suspension has high viscosity. It should be noted that a stirring operation after the dispensing step, which may be conventionally performed so as to achieve the uniformity of the cells (for example, stirring by repeated aspirating and dispensing operations in the culture vessel using the dispensing device), may not be performed in the present method of seeding cells.

**In** one embodiment, the present method of seeding cells may further comprise a step of stirring the cell suspension prior to the aspirating step. This stirring step may preferably be performed, particularly when cells which tend to easily sediment in the cell suspension. The stirring of the cell suspension may be performed by a stirring method commonly performed in the field of cell culturing, including repeated aspirating and dispensing operations using the dispensing device, or shaking.

In a preferred embodiment, the present method of seeding cells may further comprise a step of stirring the cell suspension prior to the aspirating step, wherein the aspirating step and the dispensing step are each performed once or multiple times after the stirring step. When the aspirating step and the dispensing step are each performed multiple times, it may be preferable to perform the stirring step before the first aspirating and dispensing steps. However, in some cases, such as in a case where cells which sediment quickly, it is assumed that the stirring step may be performed multiple times prior to each aspirating and dispensing step. In one embodiment, after the stirring step, the aspirating step and the dispensing step may be completed before the cells in the cell suspension sediment. Further, when multiple culture vessels, such as a multi-well plate, are used, the aspirating step and the dispensing step can be continuously performed on each (or some) of the culture vessels after the step of stirring the cell suspension is performed. For example, after performing the step of stirring the cell suspension, the aspirating step and the dispensing step may be performed on two to twelve culture vessels, such as two to six culture vessels. Alternatively, before performing the aspirating step and the dispensing step on each culture vessel, the stirring step may be performed on each culture vessel (that is, the stirring step, the aspirating step, and the dispensing step are performed on each culture vessel).

The volume of the cell suspension to be prepared can be suitably determined by a person skilled in the art by taking into consideration both the total volume of the cell suspension to be dispensed into the culture vessel and an appropriate volume at which the cells can be stirred uniformly during the stirring step prior to the aspirating step.

In one embodiment, the present method of seeding cells may be a method of seeding cells using an automated dispensing device, the method comprising the steps of:
aspirating a cell suspension using the dispensing device;
dispensing the cell suspension onto a single fixed point in a culture vessel using the dispensing device; and
leaving the culture vessel to sit without shaking after the dispensing step, thereby allowing the cells to adhere to the culture vessel.

**In** one embodiment, the present method of seeding cells may be a method of seeding cells using an automated dispensing device, the method comprising the steps of:
aspirating a cell suspension using the dispensing device;
dispensing the cell suspension onto a single fixed point in a culture vessel using the dispensing device; and
leaving the culture vessel to sit without shaking after the dispensing step, thereby allowing the cells to adhere to the culture vessel,
wherein the single fixed point in the culture vessel is in a center portion of a bottom surface of the culture vessel, and
wherein the culture vessel is a flat-bottomed (for example, a quadrangular flat-bottomed) culture vessel.

**In** one embodiment, the present method of seeding cells may be a method of seeding cells using an automated dispensing device, the method comprising the steps of:
aspirating a cell suspension using the dispensing device;
dispensing the cell suspension onto a single fixed point in a culture vessel using the dispensing device; and
leaving the culture vessel to sit without shaking after the dispensing step, thereby allowing the cells to adhere to the culture vessel,
wherein the single fixed point in the culture vessel is in a center portion of a bottom surface of the culture vessel,
wherein the culture vessel is a flat-bottomed (for example, a quadrangular flat-bottomed) culture vessel, and
wherein the cells are stem cells, preferably iPS cells.

In one embodiment, the present method of seeding cells may be a method of seeding cells using an automated dispensing device, the method comprising the steps of:
aspirating a cell suspension using the dispensing device;
dispensing the cell suspension onto a single fixed point in a culture vessel using the dispensing device; and
leaving the culture vessel to sit without shaking after the dispensing step, thereby allowing the cells to adhere to the culture vessel,
wherein the single fixed point in the culture vessel is in a center portion of a bottom surface of the culture vessel,
wherein the culture vessel is a flat-bottomed (for example, a quadrangular flat-bottomed) culture vessel (for example, a well of a 1-well plate) having a culture surface area of 3 to 500 cm² (for example, 3 to 200 cm², 3 to 100 cm², 9 to 500 cm², 9 to 200 cm², 9 to 100 cm², 20 to 500 cm², 20 to 200 cm², 20 to 100 cm², 40 to 500 cm², 40 to 200 cm², 40 to 100 cm², 60 to 500 cm², 60 to 200 cm², 60 to 150 cm², 60 to 140 cm², 60 to 120 cm², 60 to 100 cm², 70 to 500 cm², 70 to 200 cm², 70 to 150 cm², 70 to 140 cm², 70 to 120 cm², 70 to 100 cm², 80 to 500 cm², 80 to 200 cm², 80 to 150 cm², 80 to 140 cm², 80 to 100 cm², 90 to 500 cm², 90 to 200 cm², 90 to 150 cm², 90 to 140 cm², 90 to 100 cm², or 95 cm²), and
wherein the cells are stem cells, preferably iPS cells.

In one embodiment, the present method of seeding cells may be a method of seeding cells using an automated dispensing device, the method comprising the steps of:
aspirating a cell suspension using the dispensing device;
dispensing the cell suspension onto a single fixed point in a culture vessel using the dispensing device; and
leaving the culture vessel to sit without shaking after the dispensing step, thereby allowing the cells to adhere to the culture vessel,
wherein the single fixed point in the culture vessel is in a center portion of a bottom surface of the culture vessel,
wherein the culture vessel is a quadrangular flat-bottomed well of a 1-well plate having a culture surface area of 60 to 150 cm² (for example, 60 to 120 cm², 60 to 100 cm², 70 to 150 cm², 70 to 120 cm², or 70 to 100 cm²), and
wherein the cells are iPS cells.

### (Cell Seeding Device)

**In** one aspect, the present invention provides a cell seeding device, comprising:
a dispensing device;
a cell suspension holding part;
a culture vessel holding part; and
a control unit,
wherein the control unit is configured to control such that:
   a cell suspension held at the cell suspension holding part is aspirated by the dispensing device; and
   the cell suspension aspirated by the dispensing device is dispensed onto a single fixed point in a culture vessel held at the culture vessel holding part.

The present cell seeding device may further comprise a mechanism for aligning the center portion of the culture vessel in the culture vessel holding part, in order to ensure dispensing from the dispensing device to the single fixed point in the culture vessel.

The control unit may be configured to control such that the cell suspension held at the cell suspension holding part is stirred. For example, it may be configured to control the dispensing device to perform aspirating and dispensing operations in the cell suspension held at the cell suspension holding part.

**In** order to add an additional component and/or liquid to the cell suspension held at the cell suspension holding part and/or to the culture vessel held at the culture vessel holding part, the cell seeding device may further comprise an additional component and/or liquid holding part.

Further, the present cell seeding device may also comprise a device for observing the culture vessel held at the culture vessel holding part (for example, a light source, an optical detection system, an image acquisition unit, or an image analysis unit).

The control unit may be configured to control at least one of the following: the aspiration and the dispensing of the cell suspension by the dispensing device (including the dispensing speed, the number of dispensing operations, and dispensing to the single point in the culture vessel); the aligning of the culture vessel; the stirring of the cell suspension; the shaking of the culture vessel; and the addition of the additional component and/or liquid to the cell suspension and/or to the culture vessel. For example, the control unit may be configured to control the dispensing device to dispense the cell suspension onto the single fixed point in the culture vessel under suitable dispensing conditions (such as the dispensing volume and the dispensing speed) based on inputted dispensing conditions or based on inputted information, such as the type of the cells and the concentration of the cell suspension. The control unit may employ any control means known in the field of the art, and may be, for example, a computer.

As described above, the method and the device for seeding cells according to the present invention may enable uniform seeding of cells in a culture vessel.

### (Program for Seeding Cells)

In one aspect, the present invention provides a program for seeding cells using a dispensing device, the program being configured to cause the dispensing device to perform the steps of:
aspirating a cell suspension held at a cell suspension holding part; and
dispensing the cell suspension onto a single fixed point in a culture vessel held at a culture vessel holding part.

In one embodiment, the program according to the present invention may cause the dispensing device to perform each of the aspirating step and the dispensing step once or multiple times (for example, two to four times). In one embodiment, the program according to the present invention may cause the dispensing device to further perform a step of stirring the cell suspension prior to the aspirating step. For example, after the stirring step, the aspirating step and the dispensing step may preferably be completed before the cells in the cell suspension sediment. In one embodiment, according to the program of the present invention, the single fixed point in the culture vessel may be set within a center portion of a bottom surface of the culture vessel.

In addition to causing the dispensing device to aspirate the cell suspension and dispense the cell suspension into the culture vessel, the content of the control by the program may include causing the dispensing device to stir the cell suspension; causing the control unit to calculate an appropriate cell dispensing speed based on a factor such as the type of cells, the cell concentration of the cell suspension, and the size of the culture vessel; causing an observation unit to observe the number, the state, and/or the morphology of the cells; and causing an output unit to display the observation results. In such as case, it may also be possible to use reference data stored in advance in a storage unit (such as appropriate seeding conditions based on the factor such as the type of the cells, the cell concentration of the cell suspension, and the size of the culture vessel). Further, the reference data may be updated by transmitting and receiving information over an external network. It should be noted that all functional controls of the dispensing device and the like can be implemented in software by a processor interpreting and executing a program stored in a memory of the control unit. Information on each function, such as a program and a database, may be stored in a recording device, such as a memory, a hard disk, and an SSD (Solid State Drives), or in a recording medium, such as an IC card, an SD card, and a DVD.

When the program according to the present invention is implemented on a computer, for example, the following steps may be executed:
(S0) information, such as the type of the cell to be seeded, the concentration of the cell suspension, and the size of the culture vessel, may be inputted (for example, by user input or by selecting a selection panel);
(S1) based on the inputted information, the dispensing device may be made stir the cell suspension held at the cell suspension holding part, if necessary;
(S2) based on the inputted information, the dispensing device may be made aspirate an appropriate volume of the cell suspension held at the cell suspension holding part;
(S3) based on the inputted information, the dispensing device may be made dispense the cell suspension onto the single fixed point in the culture vessel held at the culture vessel holding part at an appropriate volume and speed; and
(S4) if necessary, the observation unit may be made observe the number, the state, and/or the morphology of the cells in the culture vessel, and if necessary, the output unit may be made display the observation results.

### (Method of Dispensing Cell Suspension)

In another aspect, the present invention provides a method of dispensing a cell suspension using a dispensing device, the method comprising the steps of aspirating the cell suspension using the dispensing device; and dispensing the cell suspension onto a single fixed point in a culture vessel using the dispensing device. Each of these steps can be carried out as described above. As used herein, the term "dispensing" refers to the act of distributing a certain amount of cells on a culture vessel or in a culture solution in the culture vessel.

### (Method of Culturing Cells and Cell Culturing System)

In one aspect, the present invention provides a method of culturing cells in a culture vessel, the method comprising the steps of:
seeding cells into the culture vessel by the method as described herein; and
culturing the cells in the culture vessel.

The step of culturing cells may be performed by a method suitable for the type of the cells used and the purpose of the culturing (for example, proliferation, differentiation, and the like). Culturing conditions including the manner of culturing (for example, static culturing, shaking culturing, and the like), the temperature, the pH, the presence or absence of aeration, and the culturing period can be suitably set by a person skilled in the art. If necessary, after the cell seeding step, a culture medium, medium components, nutrient components, or the like may be added to the culture vessel, or the culture medium may be replaced. For example, the culturing step may be performed under conditions where static culturing or shaking culturing is performed at 32 to 37°C, with a pH around 4.0 to 8.0, for an appropriate period. If the pH changes along the cell culturing, it may be adjusted using an inorganic or organic acid, an alkaline solution, or the like.

The number, the state, and/or the morphology of the cells may be checked by visual observation or by image capturing before, during, and/or after the cell culturing step, and if necessary, repetition or termination of the cell culturing may be determined.

The present method of culturing cells may further comprise a step of transferring the culture vessel to an incubator. In such a case, the step of culturing the cells may be performed in the culture vessel inside the incubator.

The present method of culturing cells may further comprise a step of inducing differentiation of the cells. If the cells are progenitor cells or stem cells, they may be differentiated into target cells by applying an appropriate treatment for inducing the differentiation. The differentiation inducing treatment can be performed by addition of a differentiation inducing factor to the culture medium, induction of expression of a specific gene in the cells, contact of the cells with other cells, or the like, and may vary depending on the type of the cells before inducing the differentiation and the type of target differentiated cells. A person skilled in the art can select an appropriate differentiation inducing treatment based on common technical knowledge in the field of the art, and various kits for inducing differentiation are also commercially available.

The differentiation inducing factor may not be particularly limited as long as it is commonly used in the field of the art, and examples of the differentiation inducing factor may include growth factors (such as vascular endothelial growth factors (VEGFs), basic fibroblast growth factors (bFGFs), bone morphogenetic factor 4, transforming growth factor-β (TGF-β) superfamily, insulin-like growth factors (IGFs), epidermal growth factors (EGFs), and nerve growth factor (NGFs)), horse serum (5% or less), Wnt signal activating factors (such as CHIR99021), and Wnt signal inhibiting factors (such as IWR-1, IWP-2, XAV939, and Dkk-1). These differentiation inducing factors may either be used alone or in combination. A person skilled in the art can suitably select a factor based on the type of the cells used and the type of the target differentiated cells. Alternatively or additionally, expression of a specific gene may be induced in the cells to induce differentiation of the cells. For example, the expression of a gene of a differentiation inducing factor may be induced in the cells.

The culture medium to be used for inducing differentiation may also not be particularly limited as long as it is a basal culture medium commonly used in the field of the art. Examples of such a culture medium may include Eagle's Minimum Essential Medium (MEM), Dulbecco's Modified Eagle Medium (DMEM), α-Modified Eagle's Minimum Essential Medium (α-MEM), StemFit medium, Glasgow's MEM (GMEM), RPMI 1640 medium, Ham's F12 medium, MCDB medium, Williams' E medium, Mesenchymal Stem Cell Basal Medium (MSCBM), and Essential 6 (E6) medium. If necessary, these culture media may be supplemented with amino acids, inorganic salts, vitamins, antibiotics, and the like.

The conditions for the cell culturing during induction of differentiation may be general conditions for culturing animal cells. For example, the cells can be cultured at 32 to 37°C, with a CO₂ concentration of 1 to 10% and an O₂ concentration of 2 to 21% (for example, approximately 21% (an atmospheric oxygen concentration)), and at a pH of around 4.0 to 8.0, over an appropriate period of time.

In one embodiment, the cells may be induced to differentiate by performing one or multiple differentiation inducing steps (for example, culturing cells in a culture medium containing one or multiple differentiation inducing factors). Regarding the differentiation inducing step, the same differentiation inducing step may be performed once or multiple times, or different differentiation inducing steps may be performed multiple times. For example, when the cells are cultured in a culture medium containing a differentiation inducing factor, the cell culturing may either be performed once or multiple times in a culture medium containing the same differentiation inducing factor, or performed multiple times in culture media containing different differentiation inducing factors. For example, stem cells may be cultured in a culture medium containing a differentiation inducing factor to induce the cells to differentiate into mesenchymal stem cells, ectodermal cells, mesodermal cells, or endodermal cells. Subsequently, these cells may be cultured in a culture medium containing other differentiation inducing factor so as to be further differentiated into the target differentiated cells or progenitor cells thereof. The progenitor cells may further be induced to differentiate into the target differentiated cells by culturing them in a culture medium containing another differentiation inducing factor.

When stem cells (for example, iPS cells) are used as cells before inducing differentiation, the stem cells can be induced to differentiate into any cell, including, for example, mesenchymal stem cells, ectodermal cells, mesodermal cells, endodermal cells, fibroblasts, myoblasts, cardiac muscle cells, adipocytes, hepatic cells, renal cells, pancreatic cells, mammary gland cells, corneal cells, endothelial cells, epithelial cells, epidermal cells, skeletal muscle cells, smooth muscle cells, neural cells, glial cells, dendritic cells, bone marrow cells, chondrocytes, osteoblasts, osteoclasts, osteocytes, and the like. Alternatively, when specific progenitor cells are used, the progenitor cells can be induced to differentiate into the target cells.

After the differentiation inducing step, it can be confirmed whether or not the cells have differentiated into the target cells by using a common method, such as immunostaining. For example, when fluorescence microscopy (fluorescence immunostaining) using fluorescencelabeled antibodies is used, antibodies which recognize a marker protein specific to the differentiated cells may be used as the antibodies for the fluorescence immunostaining. For example, in order to detect differentiated skeletal muscle cells, immunostaining using antibodies which recognize myosin heavy chain (MyHC) can be used.

The present method of culturing cells may further comprise a step of collecting the cultured cells or differentiated cells from the culture vessel. The number, the state, and/or the morphology of the cells may be checked by visual observation or by image capturing before or after the cell collecting step.

In another aspect, the present invention provides a cell culturing system comprising:
the cell seeding device described above;
an incubator;
a transferring mechanism; and
a control unit,
wherein the control unit is configured to control such that:
   the culture vessel is transferred by the transferring mechanism from the culture vessel holding part of the cell seeding device to the incubator; and
   cells in the culture vessel are cultured in the incubator.

The cell culturing system according to the present invention may further comprise a culture vessel, an observation unit for observing the cells in the culture vessel, a user input unit, an output unit, and the like.

### (Method of Producing Cell Stock)

In another aspect, the present invention provides a method of producing a cell stock, the method comprising the steps of seeding cells into a culture vessel by the method of seeding cells as described herein; and culturing the cells in the culture vessel.

The present method of producing a cell stock may further comprise a step of transferring the culture vessel to an incubator. In such a case, the step of culturing the cells may be performed in the culture vessel inside the incubator. Further, the present method of producing a cell stock may further comprise a step of inducing differentiation of the cells. Further, the present method of producing a cell stock may further comprise a step of collecting the cultured cells or differentiated cells from the culture vessel. The number, the state, and/or the morphology of the cells may be checked by visual observation or by image capturing before or after the cell collecting step.

### Examples

While the present invention has been generally described, specific Examples are provided below so as to be referred to for a better understanding. These Examples are intended for illustrative purposes only and are not to be construed as limiting the present invention.

All cell cultures and cultures for inducing differentiation were performed at 37°C, with 5% CO₂, and at an atmospheric oxygen concentration.

### [Example 1] Cell Seeding by Double Point Dispensing

iPS cells (201B7 line; Takahashi, K. et al., Cell. 30; 131(5): 861-872) were cultured for several days on a 6-well plate (Iwaki, 3810-006) using StemFit medium (Reprocell, AK-02N; hereinafter referred to as "iPS cell culture medium") supplemented with ROCK inhibitor Y-27632 (Wako, 036-24023, at a final concentration of 10 µM) and the coating agent iMatrix-511 (Nippi, 892012, at a final concentration of 0.25 µg/cm²), to prepare a sufficient amount of cells. Y-27632 was added until the day after the cell seeding. The proliferated iPS cells were collected into a 15 mL centrifuge tube using TrypLE Select (Gibco, 12563-011) as a dissociating agent, and were washed by centrifugation. After the supernatant was removed, the cells were resuspended in an appropriate volume of StemFit culture medium. The cell suspension was counted for the number of cells, and then suitably diluted with StemFit culture medium so that the cell density was approximately 2,000 cells/cm² (approximately 1.27×10⁴ cells/mL) when seeding the cells. The resulting cell suspension was added to a 50 mL centrifuge tube.

Prior to the cell seeding, 3 mL of iPS cell culture medium and Hoechst (Invitrogen, H1399, at a final concentration of 1 µg/mL) for nuclear stain were added to a 1-well plate (Greiner, 670180; with a culture surface area of 95 cm²) as a culture vessel.

Unlike round dishes generally used in culturing cells, the 1-well plate described above has a rectangular shape suitable for automation. Therefore, in order to seed cells uniformly, it was considered that the cell suspension should be dispensed at two fixed points, that is, one center point in each of two areas separated by a straight line vertically dividing the long axis of the rectangle.

The following cell seeding operations were performed using LabDroid Maholo (Robotic Biology Institute; hereinafter, referred to as "Maholo") capable of performing automated dispensing operation and culturing cells in an incubator. In the 50 mL centrifuge tube, 5 mL of cell suspension was aspirated and dispensed repeatedly for four times, and stirring was performed so that the cell suspension was uniformly mixed. Since the liquid volume supplied by the electronic pipette of Maholo (Viaflo II; Integra, #4015) is 5 mL or less per dispensing operation, the cells were seeded into a 1-well plate under the following conditions.
(i) In order to seed the cell suspension at 15 mL/well, 3.75 mL of cell suspension was dispensed two times onto each of the two points, that is, four dispensing operations were performed in total;
(ii) Onto each of the two points, 5 mL of cell suspension was dispensed, that is, two dispensing operations were performed in total. In order to match the final liquid volume inside the culture vessel after the seeding with 18 mL as in the condition (i), the volume of the iPS cell culture medium to be previously added to the culture vessel was adjusted to 8 mL only for that case. Further, in order to match the cell density during the cell seeding with that of the condition (i), the cell suspension was suitably diluted with StemFit culture medium to 1.90 × 10⁴ cells/mL, the resulting cell suspension was added to the 50 mL centrifuge tube, and then the seeding was performed from the 50 mL centrifuge tube.

In both of the conditions (i) and (ii), the height from which the cell suspension was to be dispensed was set to +17 mm above the bottom surface of the culture vessel, and the dispensing speed was set to 890 µL/sec.

After seeding the cells, the cells were left to sit without disturbance for 10 minutes and were allowed to adhere to the culture vessel. The cells were then placed in a cell culture incubator (37°C, 5% CO₂). Since the doubling time of the iPS cells used was approximately 30 hours, it is considered that evaluation within 10 hours after the start of culture can avoid influences on the evaluation results, because it is before the proliferation of the iPS cells. Thus, the uniformity of the cell seeding density was evaluated within 10 hours after the start of culture. Imaging of the entire surface of the culture vessel (240 fields of view in total) was performed using a confocal image cytometer CQ1 (Yokogawa Electric Corporation). The number of Hoechst-positive particles (counts/field) in each field of view was measured using an analysis software Cell Pathfinder (Yokogawa Electric Corporation), and then the C. V. value and the Max/Mean value, which are indicators of the uniformity of the cell seeding density, were calculated. In the calculations of the C.V. value and the Max/Mean value, measurement values of 180 fields of view among the 240 imaged fields of view were used, excluding those of the outermost regions of the culture vessel. This is because, if those fields of view contain a region with insufficient brightness at the edge of the well, or contain the inner wall of the well, it affects accurate recognition of the cells. The C.V. value is a value for evaluating variation in the number of cells in the entire well, and is generally calculated by dividing a standard deviation (S.D.) by a mean value. A lower C.V. value means less variation. The Max/Mean value is a value obtained by dividing the maximum counts/field value (Max) by the mean value (Mean), and is a value for evaluating the extent of excess of the cell density as compared to the expected cell density, if the cell density is locally high. A lower Max/Mean value is more preferable. The uniformity of the cells in the culture vessel was also assessed using a heat map created based on the numbers of cells in the respective fields of view, in addition to the C.V value and the Max/Mean value. The heat map is a map indicating the measurement values of the respective 180 fields of view as described above by shades of color. It can be judged that, as the visual differences in the shades of color on the heat map become smaller, the distribution can be determined to be more uniform and with less local bias in the cell density.

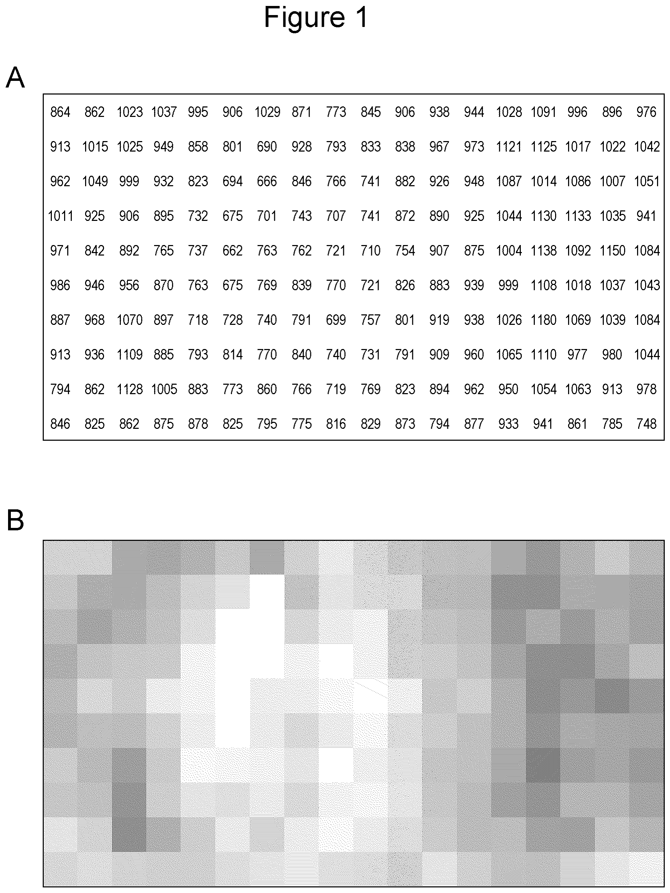

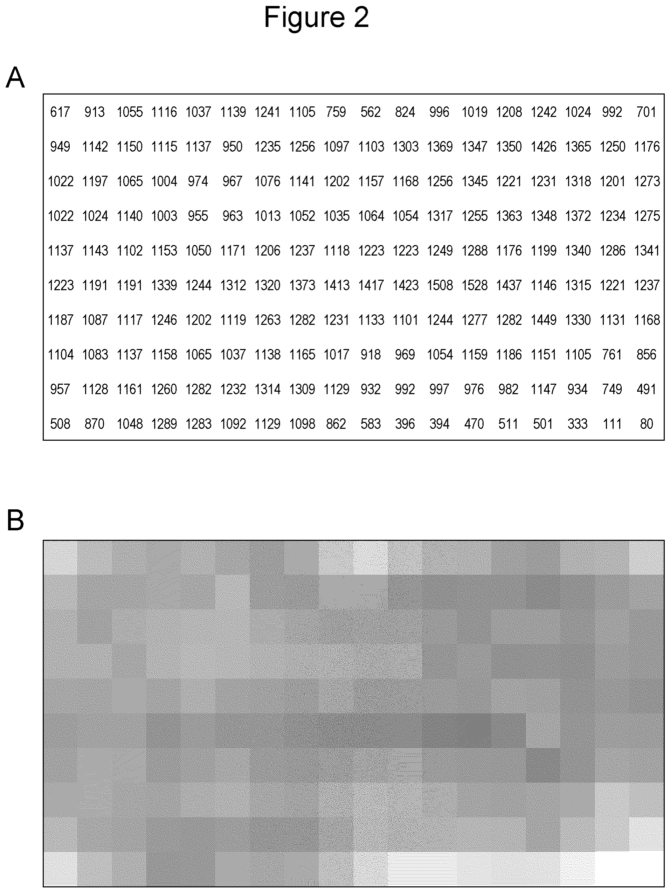

The results are shown in Table 1 below and in Figures 1 and 2. Figure 1 shows the results under the condition (i), and Figure 2 shows the results under the condition (ii). In each of Figures 1 and 2, the panel A shows the cell densities (the numbers of cells per field of view), and the panel B shows a heat map based on the cell densities.

**[Table 1]**

| Conditions | (i) | (ii) |
|---|---|---|
| Volume of iPS cell culture medium in culture vessel before dispensing | 3 mL | 8 mL |
| Dispensing method | Double point | Double point |
| Dispensing volume / total number of dispensing operations | 3.75 mL/4 times | 5 mL/2 times |
| C.V. value | 14% | 22% |
| Max/Mean value | 131% | 139% |

Under the both conditions (i) and (ii), the cells were found to be more concentrated around the two dispensing points, indicating the possibility that the cell properties may become inhomogeneous when the cells are seeded and induced to differentiate under such conditions (see Table 1 and Figures 1 to 2).

### [Example 2] Cell Seeding by Single Point Dispensing and Investigation of Whether or Not Shaking of Culture Vessel Should be Performed

Since in Example 1 the results showed that the cells were distributed more concentrated around the two dispensing points, the point onto which the cell suspension was to be dispensed was fixed to a single point at the center of the culture vessel. In addition, it was investigated whether or not shaking of the culture vessel should be performed after dispensing the cell suspension, expecting that shaking would contribute to the uniform distribution of the cells.

Procedures from the preparation of the iPS cells to the suspension of the cell suspension using Maholo were performed by the methods described in Example 1. Then, 5 mL of the cell suspension was aspirated and dispensed onto the single point at the center of the culture vessel using Mahalo. This dispensing operation was performed three times, that is, 15 mL of the cell suspension was dispensed into the culture vessel. The dispensing height and dispensing speed of the cell suspension were the same as in Example 1. The cells were seeded into three culture vessels under exactly the same conditions.

Immediately after dispensing the cell suspension, two out of the three culture vessels were each moved horizontally in a left-right direction at a certain distance in each direction (with an amplitude of approximately 10 cm or less to each direction) at a speed of 90 mm/sec, and then returned to the original position, followed by a horizontal movement in a front-back direction at a certain distance in each direction (with an amplitude of approximately 10 cm or less to each direction) and returning to the original position. This series of movements constitute one shaking operation, and the culture vessel was shaken 10 or 20 times. In the present Example, "the left-right direction" of the shaking refers to a direction along the axis parallel to the short side of the 1-well plate, and "the front-back direction" refers to a direction along the axis parallel to the long side of the 1-well plate. The three culture vessels were then left to sit without disturbance for 10 minutes to allow the cells to adhere to the culture vessel, and then placed in a cell culture incubator (37°C, 5% CO₂). The uniformity of the cell seeding density was evaluated as in Example 1 within 10 hours after the start of culture.

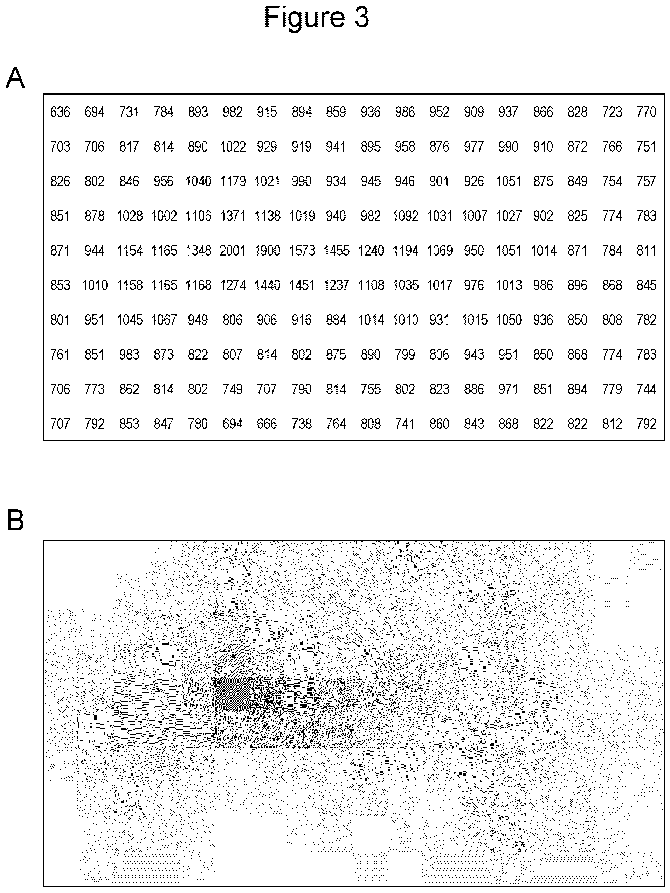

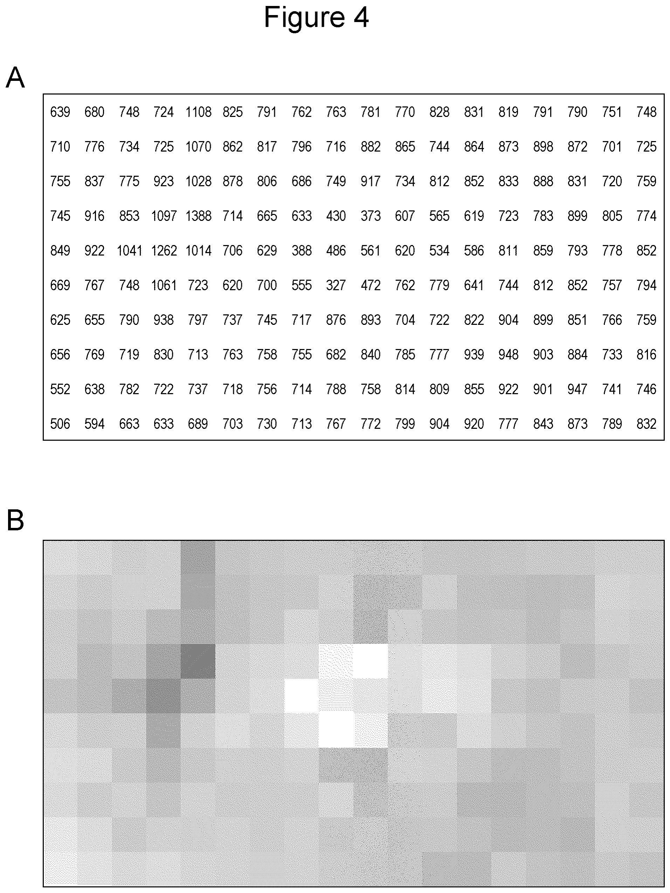

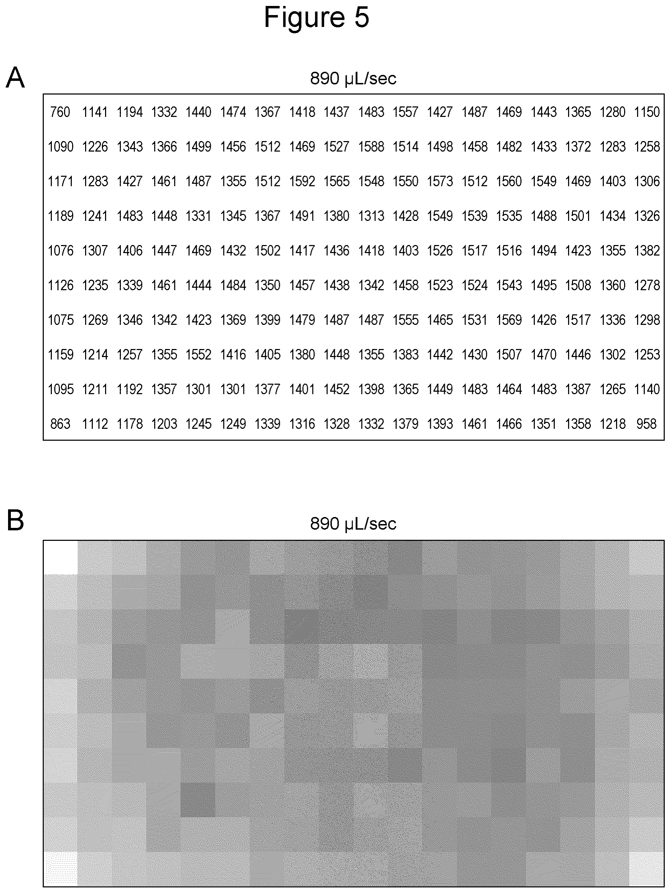

The results are shown in Table 2 below and in Figures 3 to 5. Figure 3 shows the results when 10 shaking operations were performed, Figure 4 shows the results when 20 shaking operations were performed, and Figure 5 shows the results when no shaking was performed. In each of Figures 3 to 5, the panel A shows the cell densities (the numbers of cells per field of view), and the panel B shows a heat map based on the cell densities.

**[Table 2]**

| Conditions | Shaking 10 times | Shaking 20 times | No shaking |
|---|---|---|---|
| Volume of iPS cell culture medium in culture vessel before dispensing | 3 mL | 3 mL | 3 mL |
| Dispensing method | Single point | Single point | Single point |
| Dispensing volume / total number of dispensing operations | 5 mL/3 times | 5 mL/3 times | 5 mL/3 times |
| Shaking | 10 times 90 mm/sec | 20 times 90 mm/sec | 0 times |
| C. V. value | 21% | 18% | 10% |
| Max/Mean value | 216% | 179% | 115% |

The results revealed that it was possible to seed the cells more uniformly by the single point dispensing than by the double point dispensing. Further, it was revealed that it was possible to seed the cells more uniformly when the culture vessel was not shaken after the dispensing of the cell suspension (see Table 2 and Figures 3 to 5).

### [Example 3] Investigation of Stirring Method

In order to seed cells more uniformly, the stirring method of the cell suspension was investigated using Maholo. In Example 2, the cell suspension was stirred four times in the 50 mL centrifuge tube, and then 5 mL of the cell suspension was transferred from the centrifuge tube to the culture vessel (that is, aspirating and dispensing operations were performed) three times to seed the cells. In the present Example, a part of this operation was modified. After stirring the cell suspension four times in the 50 mL centrifuge tube, 5 mL of the cell suspension was transferred from the centrifuge tube to the culture vessel once, and this operation was performed three cycles to seed the cells (that is, stirring was performed in each cycle). All conditions, except for those of the stirring and transferring of the cell suspension, were the same as those in the case of no shaking in Example 2.

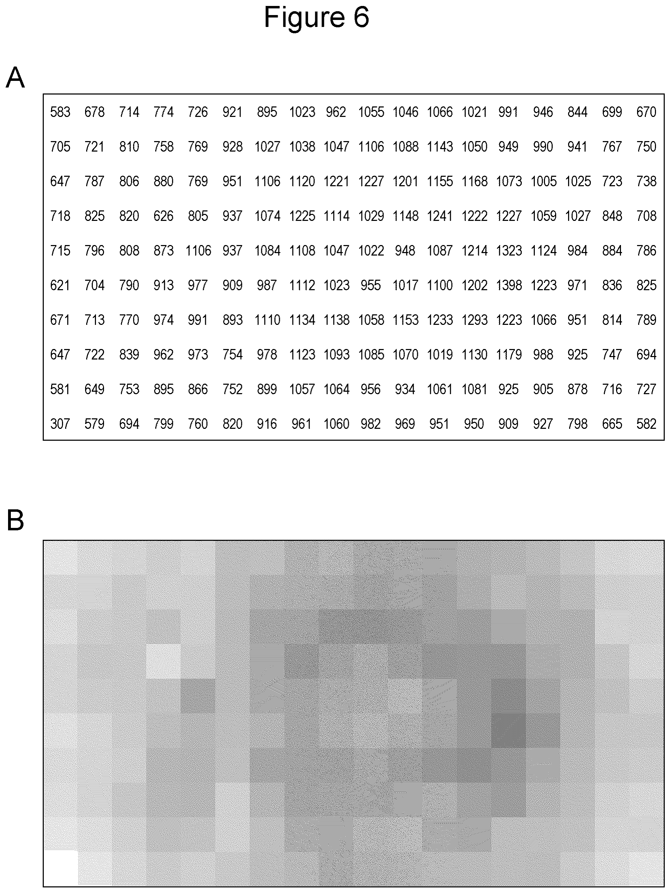

The results are shown in Figure 6. In Figure 6, the panel A shows the cell densities (the numbers of cells per field of view), and the panel B shows a heat map based on the cell densities. Further, under the condition where stirring was performed each time, the C.V. value was 19%, and the Max/Mean value was 150%. Under the condition where stirring was performed each time, although the cell distribution was uniform to an acceptable extent, there was slight bias toward the center of the culture vessel (Figure 6). Therefore, it was revealed that, while stirring may be performed each time the cell suspension is dispensed into the culture vessel, it was possible to seed the cells more uniformly when the transferring (aspirating and dispensing) operation was performed continuously after stirred once, as in Example 2.

### [Example 4] Investigation of Dispensing Speed of Cell Suspension

In view of Example 2, it was revealed that the conditions where the culture vessel was not shaken after dispensing the cell suspension were better for seeding the cells uniformly. However, it was predicted that the cell distribution around the single point at the center was dependent on the dispensing speed of the cell suspension. Lower speeds might possibly lead to distribution with more concentration at the center, while higher speeds might possibly lead to more peripheral distribution. Therefore, the speed of dispensing the cell suspension into the culture vessel was investigated using Maholo. The investigated dispensing speeds are shown in Table 3. All conditions, except for the dispensing speed, were the same as those in the case of no shaking in Example 2.

**[Table 3]**

| Dispensing speed of electronic pipet, 5000 µL (Viafloll: Integra, #4015) mounted on Maholo | |
|---|---|
| Settings | [µL/sec] |
| Speed-1 | 179 |
| Speed-2 | 358 |
| Speed-3 | 890 |
| Speed-4 | 1078 |
| Speed-5 | 1315 |
| Speed-6 | 1780 |
| Speed-7 | 2520 |
| Speed-10 | 4319 |

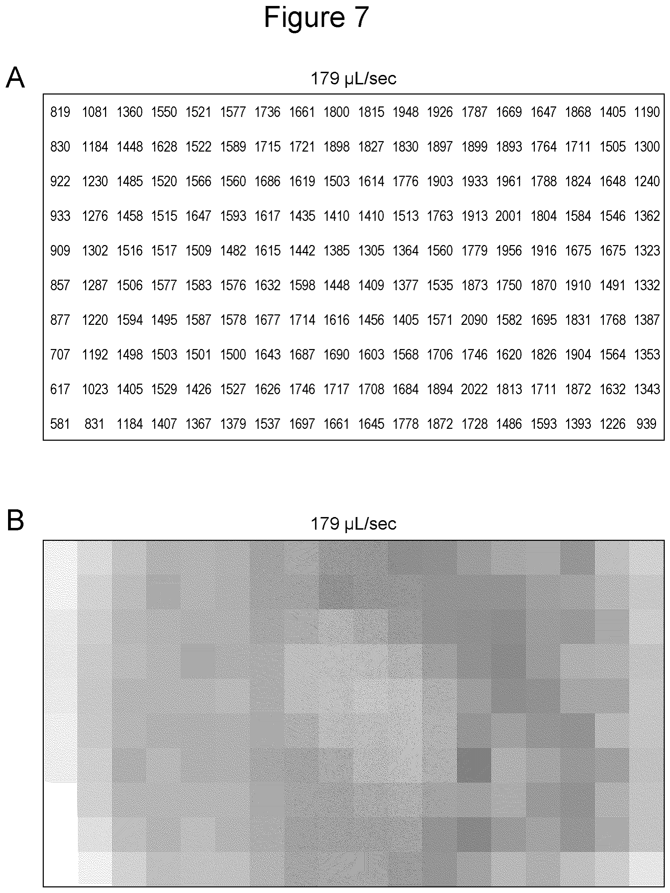

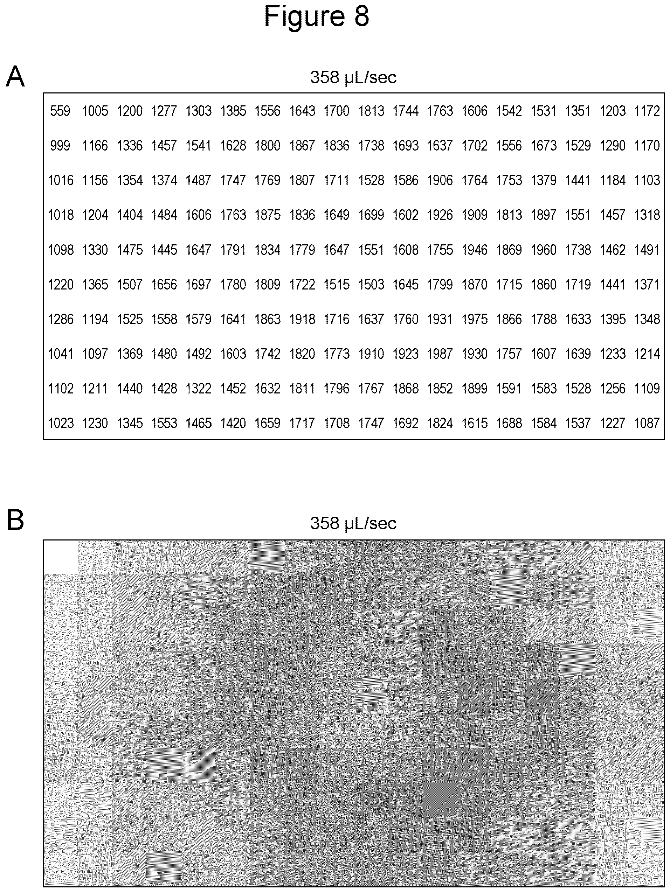

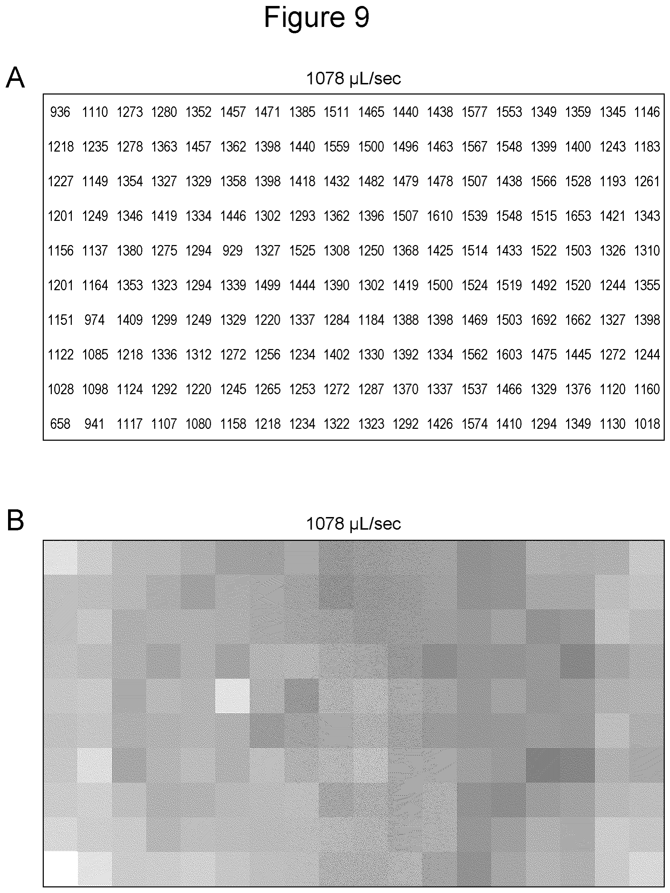

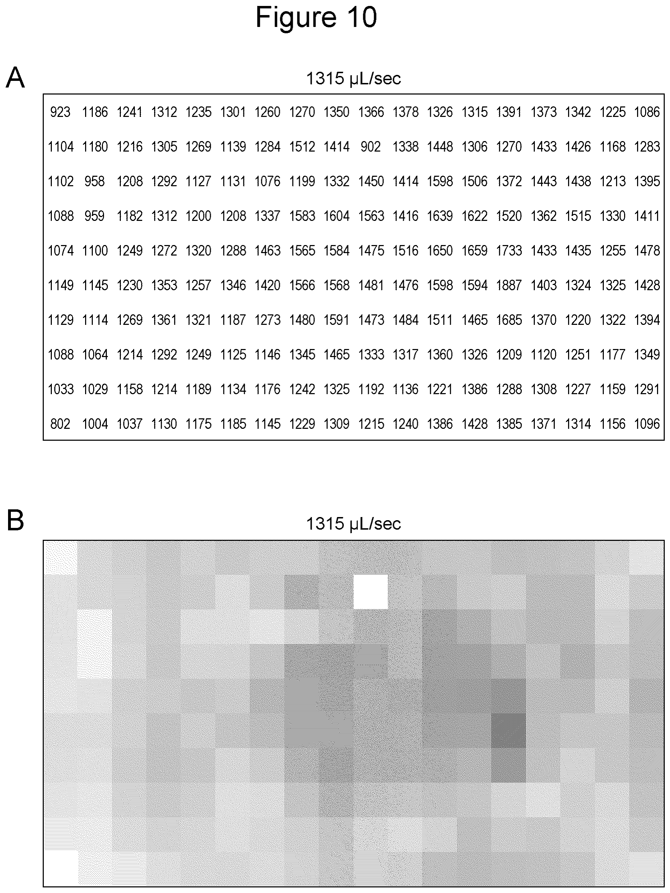

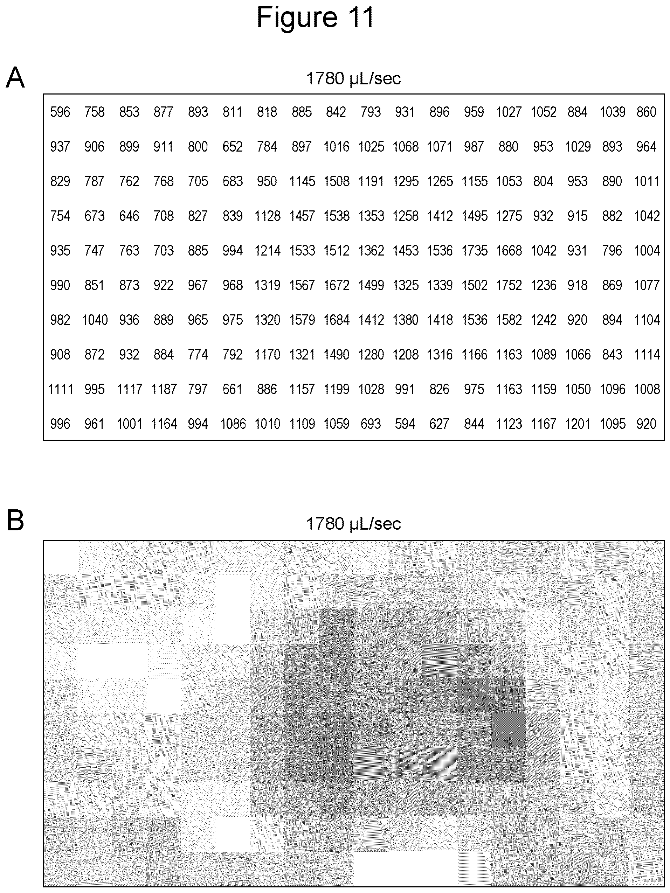

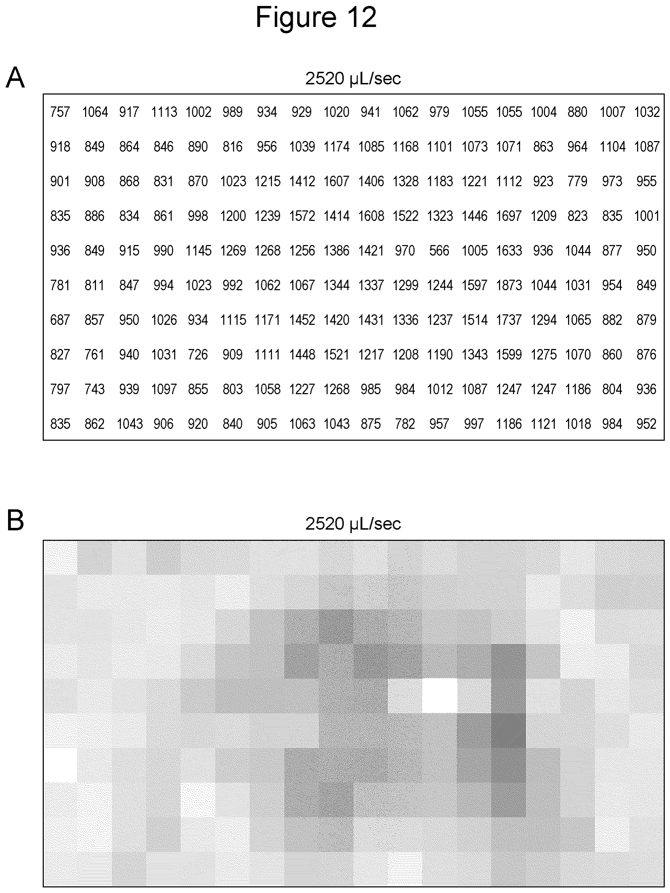

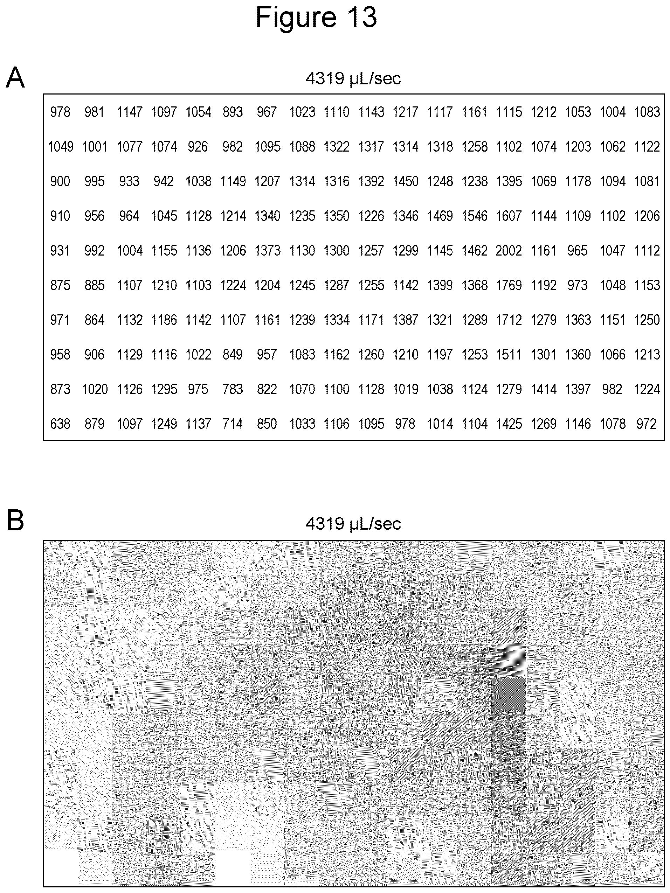

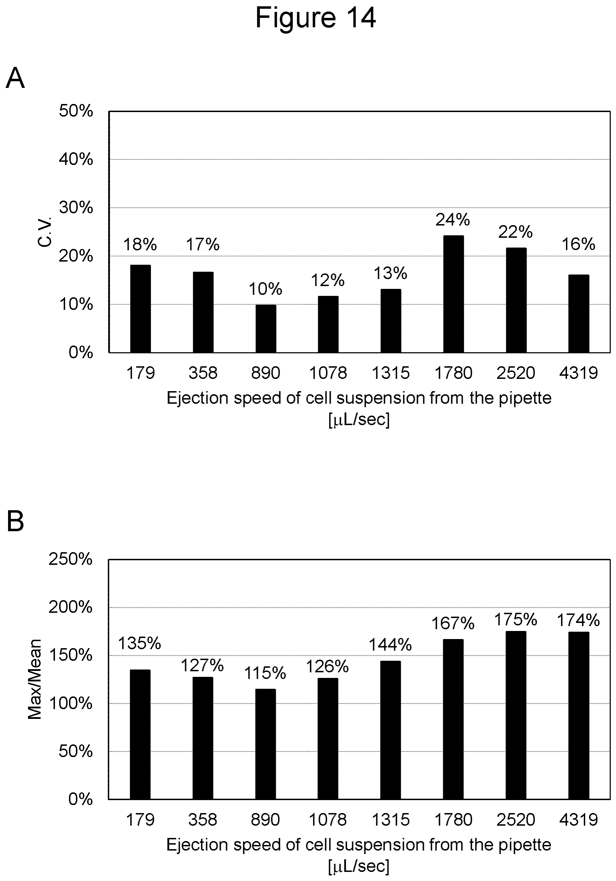

The results are shown in Figure 5 and Figures 7 to 14. In each of Figure 5 and Figures 7 to 13, the panel A shows the cell densities (the numbers of cells per field of view) at the indicated dispensing speed, and the panel B shows a heat map based on the cell densities. In Figure 14, the panels A and B respectively show the C.V. value and the Max/Mean value for each dispensing speed. The results revealed that it was possible to seed the cells uniformly at the dispensing speed within the range of 358 to 1315 µL/sec. Especially, it was revealed that it was possible to seed the cells more uniformly at the dispensing speed within the range of 890 to 1078 µL/sec (see Figure 5 and Figures 7 to 14). Therefore, it was revealed that the condition without shaking in Example 2 were optimal condition for seeding the cells uniformly.

### [Example 5] Investigation of Reproducibility of Cell Seeding

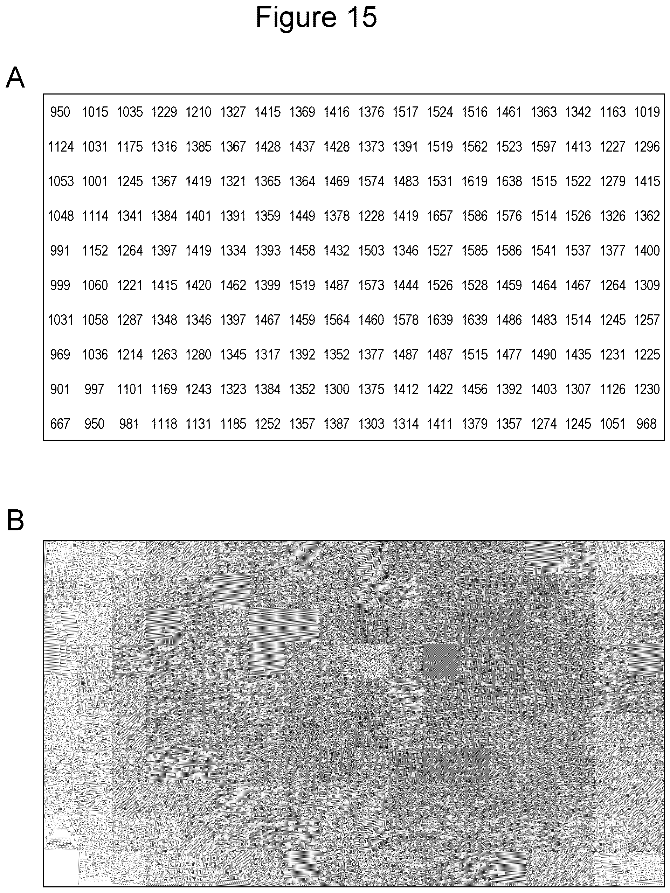

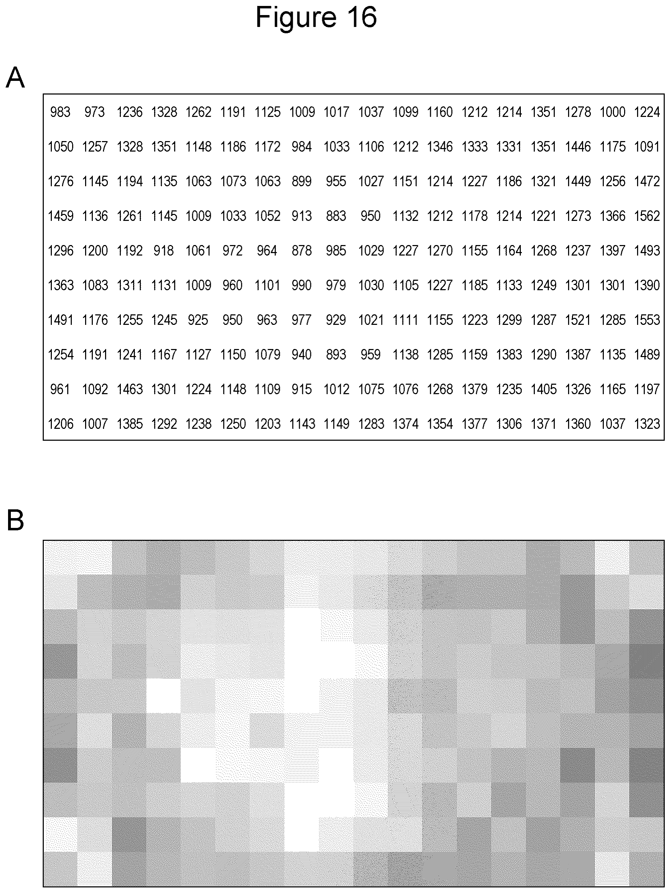

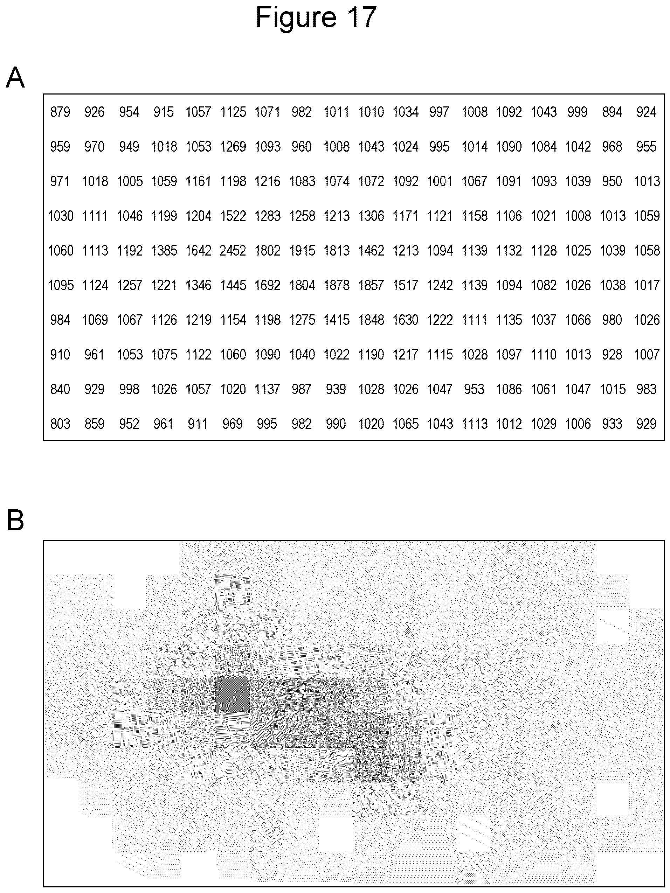

Experiments for confirming the reproducibility of Examples 1 and 2 were conducted using Maholo. The conditions and obtained results are summarized in Table 4 below, and the results are shown in Figures 15 to 17. Figure 15 shows the results under the condition where the single point dispensing was performed and shaking was not performed. Figure 16 shows the results under the condition where the double point dispensing was performed and shaking was not performed. Figure 17 shows the results under the condition where the single point dispensing was performed and shaking was performed 10 times. In each of Figures 15 to 17, the panel A shows the cell densities (the numbers of cells per field of view), and the panel B shows a heat map based on the cell densities.

**[Table 4]**

| Volume of iPS cell culture medium in culture vessel before dispensing | 3 mL | 3 mL | 3 mL |
|---|---|---|---|
| Dispensing method | Single point | Double point | Single point |
| Dispensing speed | 890 µL/sec | 890 µL/sec | 890 µL/sec |
| Dispensing volume / total number of dispensing operations | 5 mL/3 times | 3.75 mL/4 times | 5 mL/3 times |
| Shaking | 0 times | 0 times | 10 times 90 mm/sec |
| C.V. value | 13% | 13% | 20% |
| Max/Mean value | 123% | 132% | 219% |

The results confirmed that it was possible to seed the cells more uniformly when the cell suspension was dispensed onto a single point at the center of the culture vessel than when the culture vessel is divided into two areas and the cell suspension was dispensed onto a single point at the center of each of the areas (that is, two points in total). It was also confirmed that the condition without the step of shaking the culture vessel after dispensing the cell culture solution enabled seeding of the cells with higher uniformity (see Table 4 and Figures 15 to 17).

### [Example 6] Investigation of Cell Seeding Conditions Using HEK293T Cells

Using HEK293T (Thermo Scientific, HCL4517), an immortalized cell line, instead of iPS cells, it was tested whether the cells could be seeded uniformly in the culture vessel similarly applying the optimal conditions identified in the previous Examples using Maholo. In this case, the culture medium was DMEM (Sigma-Aldrich, D5796-500ML) containing 10% FBS (HyClone, SH30070.03) and 1% PenStrep (Gibco, 15070-063), and the culture vessel was a 1-well plate pre-coated with Poly-D-Lysine solution (Gibco, A3890401, at a final concentration of 4.2 µg/cm²).

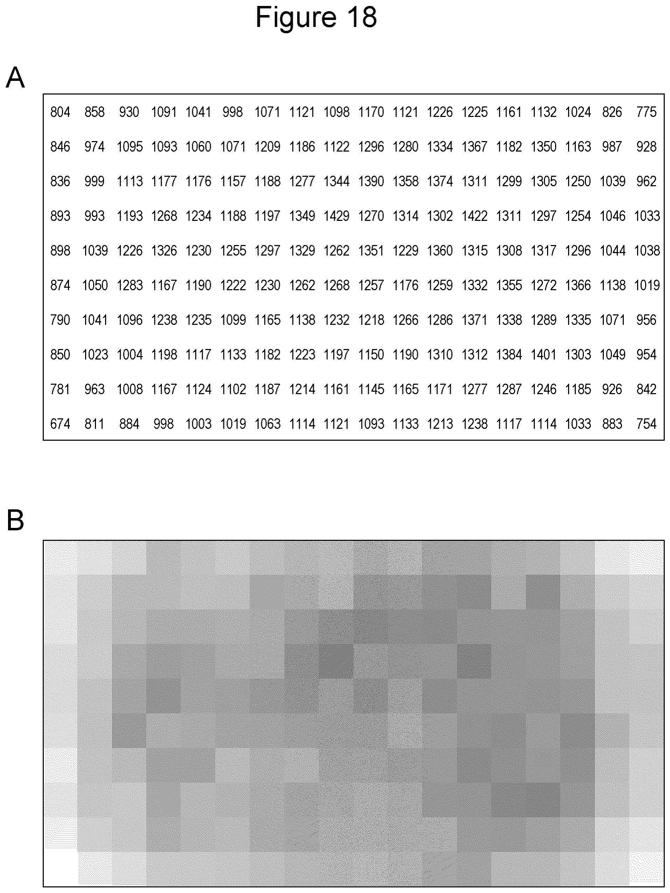

The results are shown in Figure 18. In Figure 18, the panel A shows the cell densities (the numbers of cells per field of view), and the panel B shows a heat map based on the cell densities. Further, the C.V. value was 14%, and the Max/Mean value was 124%. It was revealed that, also in the seeding of the HEK293T cells, it was possible to seed the cells uniformly under the same condition as the case without shaking in Example 2.

### [Example 7] Investigation of Homogeneity of Cells after Inducing Differentiation Using iPS Cells

It was investigated whether highly homogeneous differentiated cells could be obtained from cells seeded using the method of dispensing onto a single point at the center of the culture vessel. In the present Example, B7-MYOD-NMJ iPS cell line obtained by incorporating a doxycycline (Dox)-inducible MYOD expression vector into iPS cells (201B7) (Lin, CY. *et al.,* JCI Insight. 2019; 4(18):e124299; hereinafter, referred to as "201B7^{MYOP}") was used. MYOD has been known as a master gene for promoting induction of skeletal muscle differentiation (Davis, RL. et al., Cell. 1987; 51(6):987-1000). The addition of doxycycline (Dox) to the culture medium during the induction of differentiation of 201B7^{MYOD} cells promotes the forced expression of MYOD in 201B7^{MYOD}, enabling the differentiation of iPS cells into skeletal muscle cells for a short period.

The cells were cultured for several days in mTeSR1 culture medium (Stemcell Technologies, ST-85850) supplemented with Rock inhibitor Y-27632 (Nacalai Tesque, 18188-04, at a final concentration of 10 µM) on a culture vessel coated with Matrigel (Corning, 354230, at a final concentration of 10 µg/cm²) to obtain a sufficient amount of cells. Y-27632 was added until the day after seeding the cells.

The proliferated 201B7^{MYOD} cells were subjected to differentiation induction. For the seeding conditions for differentiation induction culture, two conditions were compared: the optimal condition in the previous Examples, where the single point dispensing was performed and shaking was not performed (hereinafter, referred to as the "single point dispensing condition"); and the condition where double point dispensing was performed and shaking was not performed (hereinafter, referred to as the "double point dispensing condition"). The culture medium for the cell seeding was Primate ES Cell culture medium (Reprocell, RCHEMD001), and the culture vessel was a 1-well plate coated with Matrigel (at a final concentration of 10 µg/cm²) and pre-filled with Primate ES Cell culture medium containing Y-27632 (at a final concentration of 10 µM). The cell density during the cell seeding was approximately 2.88 × 10⁴ cells/cm² (the cell suspension was adjusted to approximately 1.82 × 10⁵ cells/mL for the single point dispensing condition, and approximately 2.74 × 10⁵ cells/mL for the double point dispensing condition). The cells were seeded in accordance with Table 5. After the cell seeding, the cells were left to sit without disturbance for 10 minutes so as to adhere to the culture vessel, and then placed in a cell culture incubator (37°C, 5% CO₂). On the day after the seeding, the culture medium was replaced with Primate ES Cell culture medium containing 1 µg/mL of Dox (LKT Labs, D5897), and on the following day, it was replaced with a differentiation inducing culture medium. Thus, the cells were cultured for seven days after the cell seeding. The differentiation inducing culture medium was αMEM (Gibco, 12571063) containing 10% KnockOut Serum Replacement (Gibco, 10828028), 0.5% PenStrep, 1 µg/mL Dox, and 200 µM 2-mercaptoethanol (Gibco, 21985023).

The entire surface of the culture vessel was imaged to evaluate the uniformity and the homogeneity of the cells after inducing differentiation. Regarding the images, although in Examples 1 to 6 the entire surface of the culture vessel was imaged at 2 × magnification (240 fields of view in total), imaging in the present Example was performed at 4 × magnification (851 fields of view) to obtain clearer images, in order to improve the analysis accuracy. Among the 851 fields of view obtained by imaging the entire surface of the culture vessel, analysis was conducted using the measurement values of 735 fields of view, excluding those of the outermost regions of the culture vessel. In addition to the evaluation of the uniformity of the seeding density using Hoechst staining after the seeding, immunostaining using Myosin heavy chain (MyHC) as an indicator of skeletal muscle differentiation, and imaging and analysis were performed after the culturing for inducing differentiation. In the present Example, there were a very large number of cells and thus, many cell populations were adjacent to each other immediately after the seeding, which made it difficult to accurately detect the number of Hoechst particles. Thus, the uniformity after the seeding was evaluated based on Hoechst-positive area. The homogeneity of the differentiated cells was evaluated based on a value obtained by dividing MyHC-positive area by Hoechst-positive area. Immunostaining was performed using anti-MyHC antibody (eBioscience, 14-6503-82), after fixation with 4% paraformaldehyde (FUJIFILM Wako Pure Chemical, 163-20145).

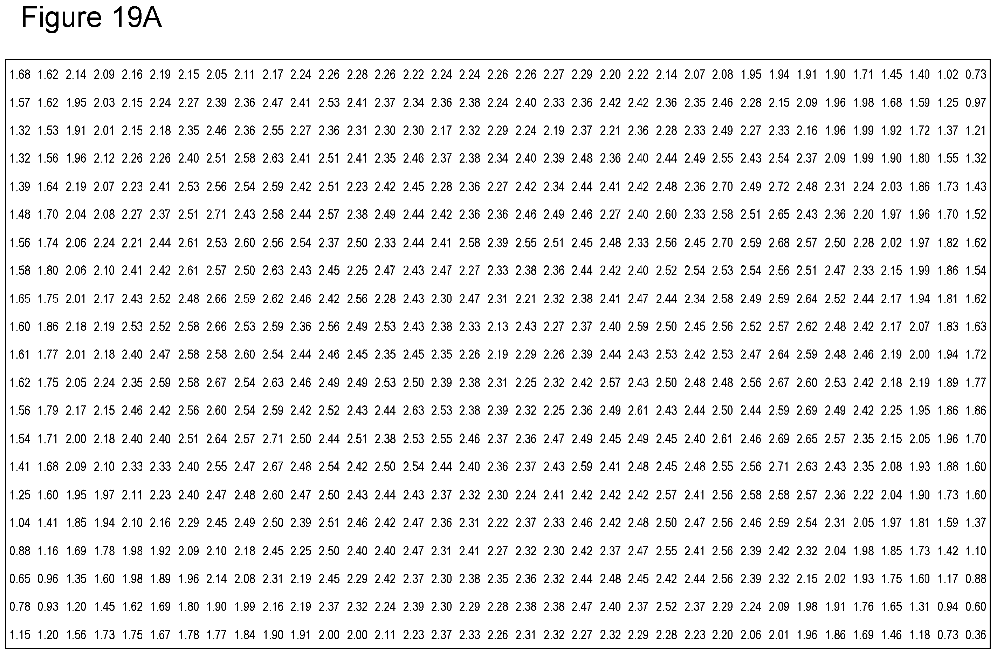

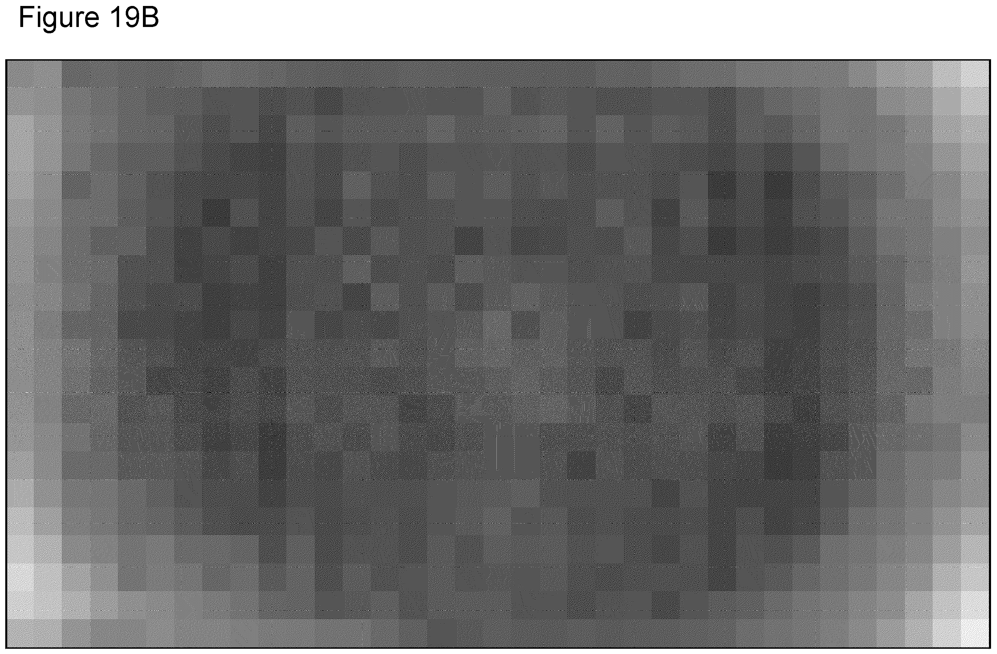

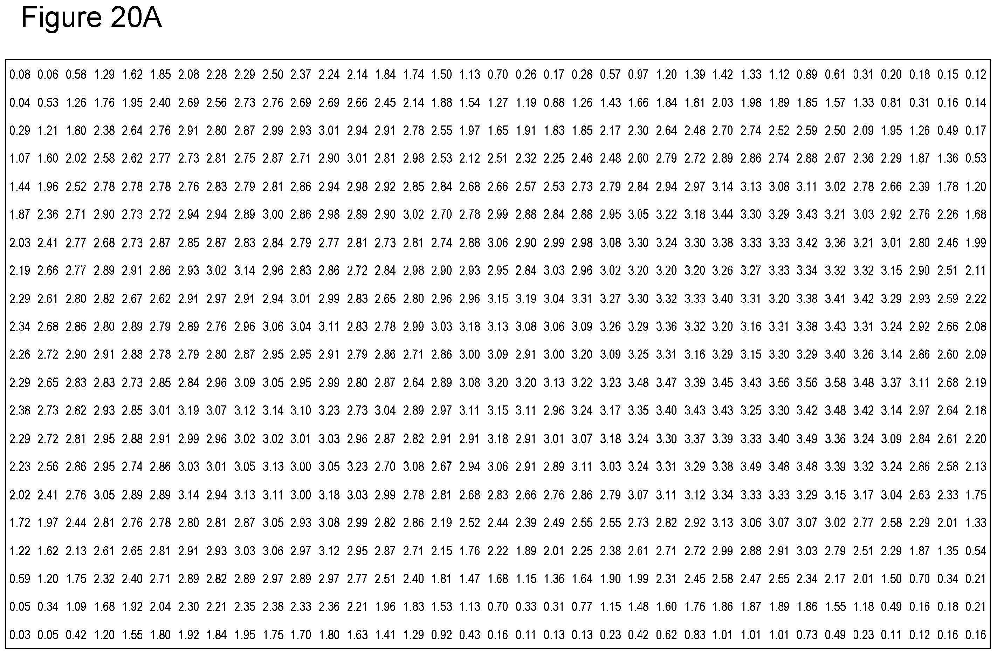

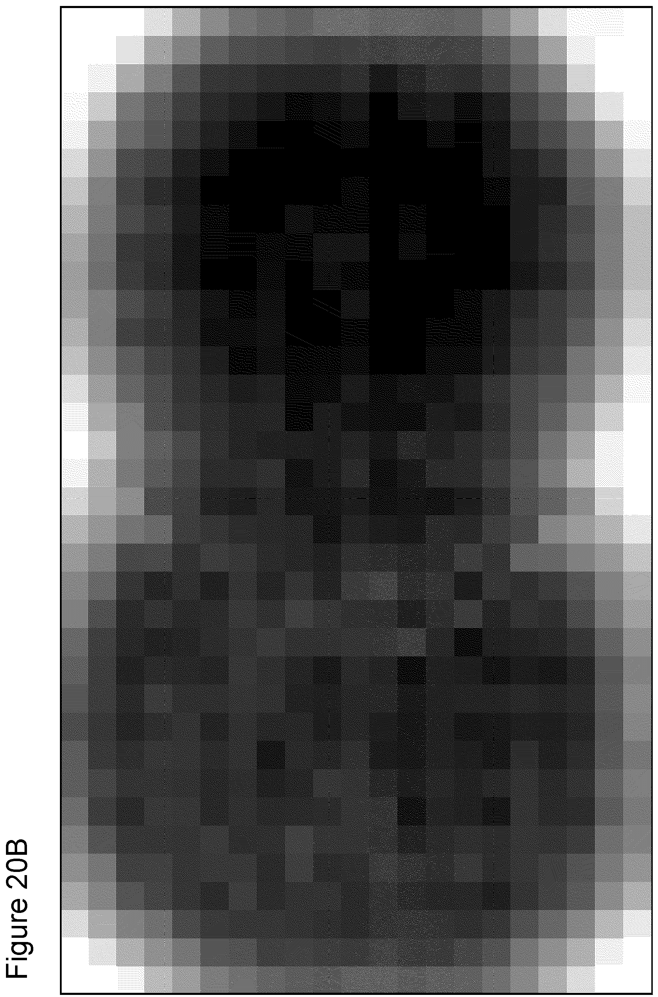

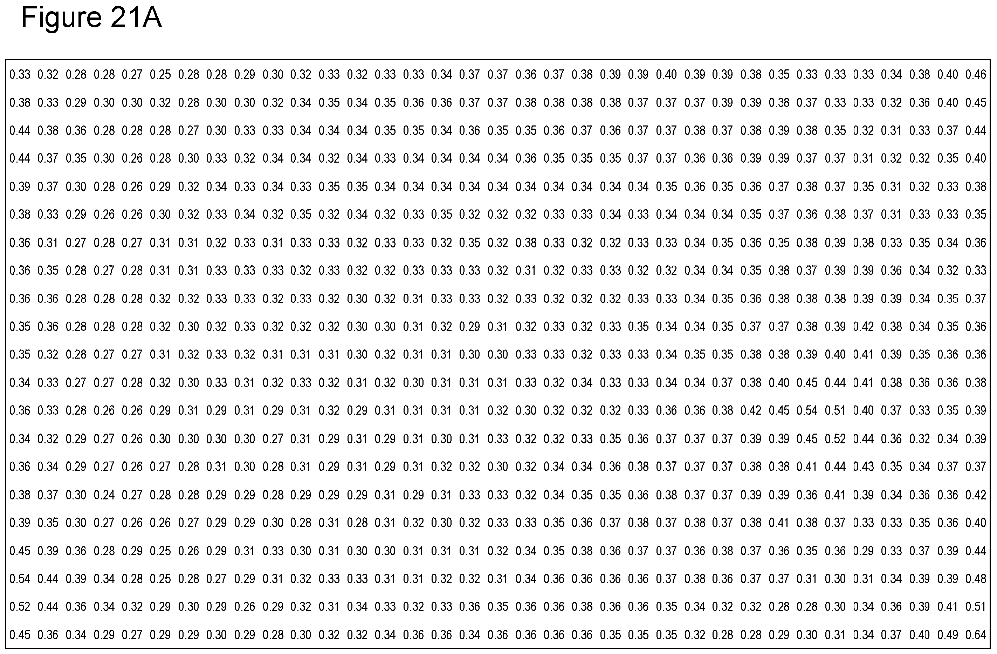

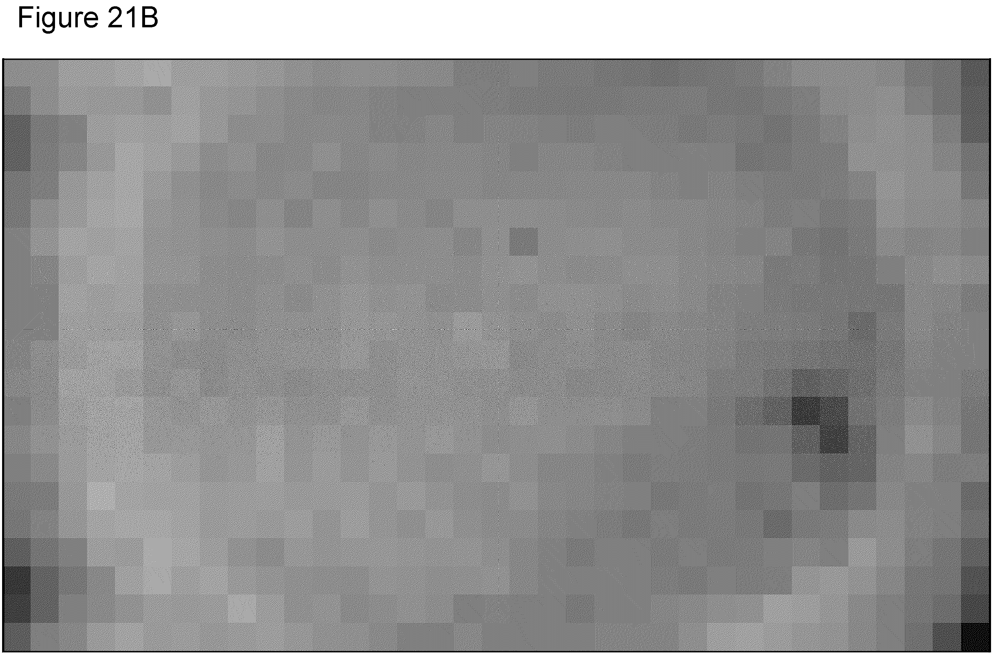

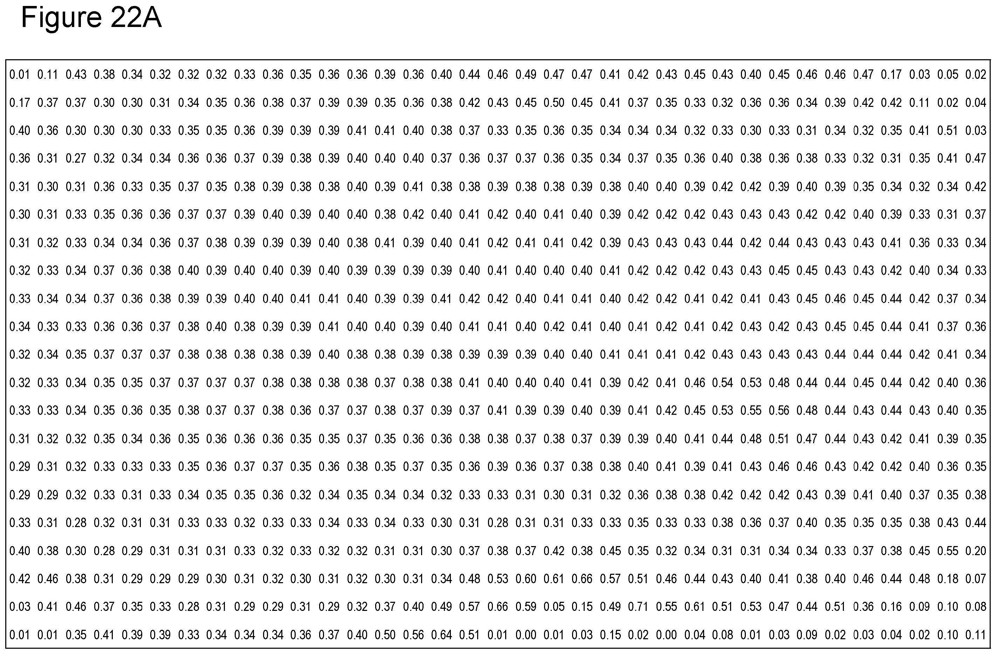

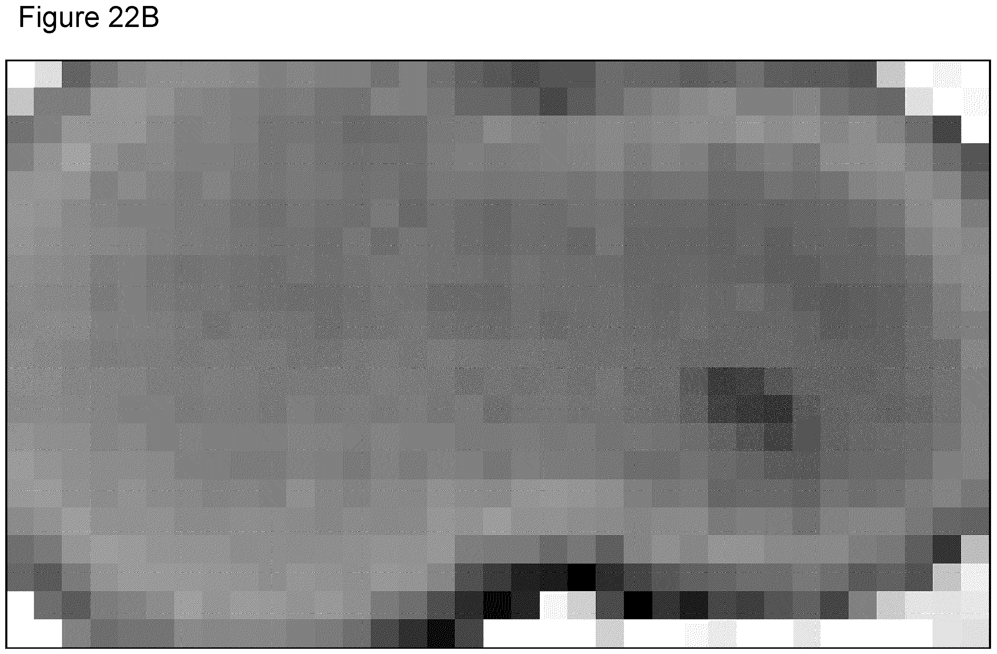

The results of evaluation of the seeding uniformity and evaluation of the homogeneity after induction of differentiation are summarized in Table 5 below and shown in Figures 19A to 22B. Figures 19A and 19B show the cell seeding density under the single point dispensing condition, Figures 20A and 20B show the cell seeding density under the double point dispensing condition, Figures 21A and 21B show MyHC expression level per cell after induction of differentiation under the single point dispensing condition, and Figures 22A and 22B show MyHC expression level per cell after induction of differentiation under the double point dispensing condition.

**[Table 5]**

| Volume of culture medium in culture vessel before dispensing | 3 mL | 8 mL |
|---|---|---|
| Dispensing method | Single point | Double point |
| Dispensing speed | 890 µL/sec | 890 µL/sec |
| Dispensing volume / total number of dispensing operations | 5 mL/3 times | 5 mL/2 times |
| Shaking | 0 times | 0 times |
| C.V. value at seeding | 17% | 34% |
| Max/Mean value at seeding | 122% | 144% |
| C.V. value after inducing differentiation | 13% | 25% |
| Max/Mean value after inducing differentiation | 188% | 193% |

Regarding the seeding uniformity, results similar to those in Examples 1 to 6 were obtained, and it was confirmed that the single point dispensing condition achieved higher uniformity (see Table 5, and Figures 19A and B and Figures 20A and B). Also under conditions with higher seeding cell densities as compared to the conditions of Examples 1 to 6, it was revealed that it was possible to seed the cells uniformly by using the single point dispensing condition. Regarding the homogeneity after induction of differentiation, darker areas on the heat maps indicate regions where MyHC-positive area is large over the number of cells, and are considered as regions with more advanced skeletal muscle differentiation than in surrounding regions. On the other hand, lighter areas indicate regions where MyHC-positive area is small over the number of cells, and are considered as regions with less advanced skeletal muscle differentiation than in surrounding regions. Thus, smaller differences in the shades of color on the heat maps can be regarded to indicate greater homogeneity of the differentiated cells. Under the double point dispensing condition, especially large differences in the shades of color on the heat map are observed near the side surface (edge) of the culture vessel, and the C.V. value was 25% (see Figures 22A and 22B). On the other hand, under the single point dispensing condition, the C.V. value was 13%, indicating that differentiated cells with higher homogeneity can be obtained by using the seeding method which achieves high uniformity (see Figures 21A and 21B).

### [Example 8] Investigation of Homogeneity of Cells after Inducing Differentiation Using C2C12 Myoblasts

In the present Example, it was investigated whether highly homogeneous differentiated cells could be obtained as in the case of iPS cells, by using C2C12 (ATCC CRL-1772) cells capable of differentiating into skeletal muscle.

Regarding the seeding conditions, the single point dispensing condition and the double point dispensing condition were compared with each other, as in Example 7. The culture medium for the cell seeding was DMEM containing 10% FBS (HyClone, SH30084.03) and 1% PenStrep, and the culture vessel was a 1-well plate pre-coated with iMatrix-511 (at a final concentration of 0.25 µg/cm²). The cell density during the cell seeding was approximately 3 × 10⁴ cells/cm² (the cell suspension was adjusted to approximately 1.90 × 10⁵ cells/mL for the single point dispensing condition, and 2.85 × 10⁵ cells/mL for the double point dispensing condition). The cells were seeded in accordance with Table 6. On the day after the seeding, the culture medium was replaced with the differentiation inducing culture medium, and differentiation was induced for about three days. The differentiation inducing culture medium was DMEM containing 2% Horse serum (Gibco, 16050-122) and 1% PenStrep. Immunostaining was performed using anti-MyHC antibody (Invitrogen, 50-6503-82), after fixation with 4% paraformaldehyde. Further, regarding evaluation of the uniformity immediately after the seeding, since single cells could be detected, the number of Hoechst-positive particles were counted as in Examples 1 to 6. The homogeneity after the differentiation was evaluated based on the value obtained by dividing MyHC-positive area by Hoechst-positive area, as in Example 7.

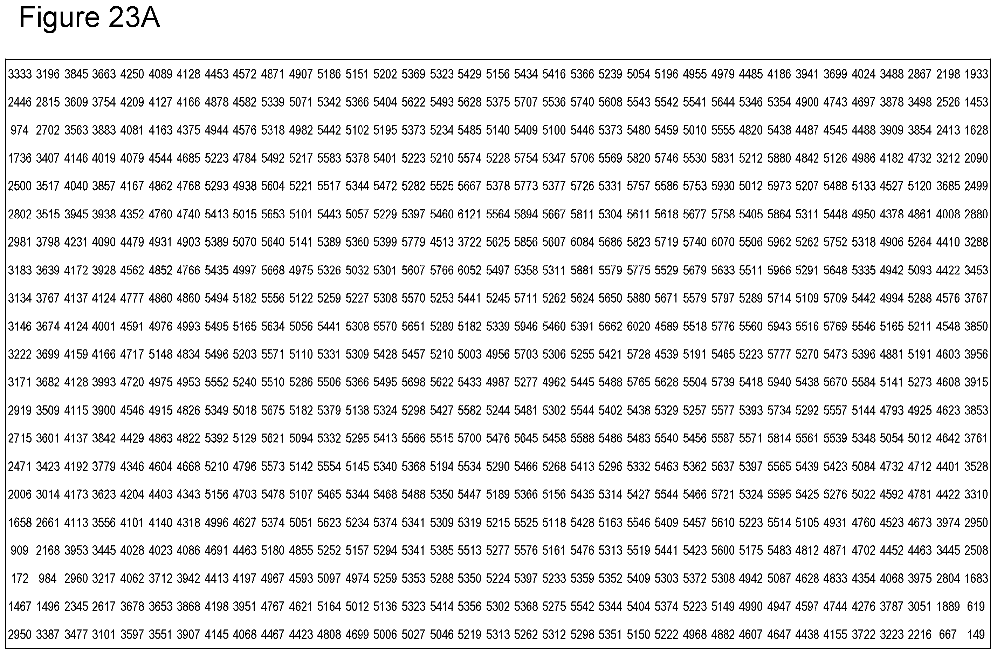

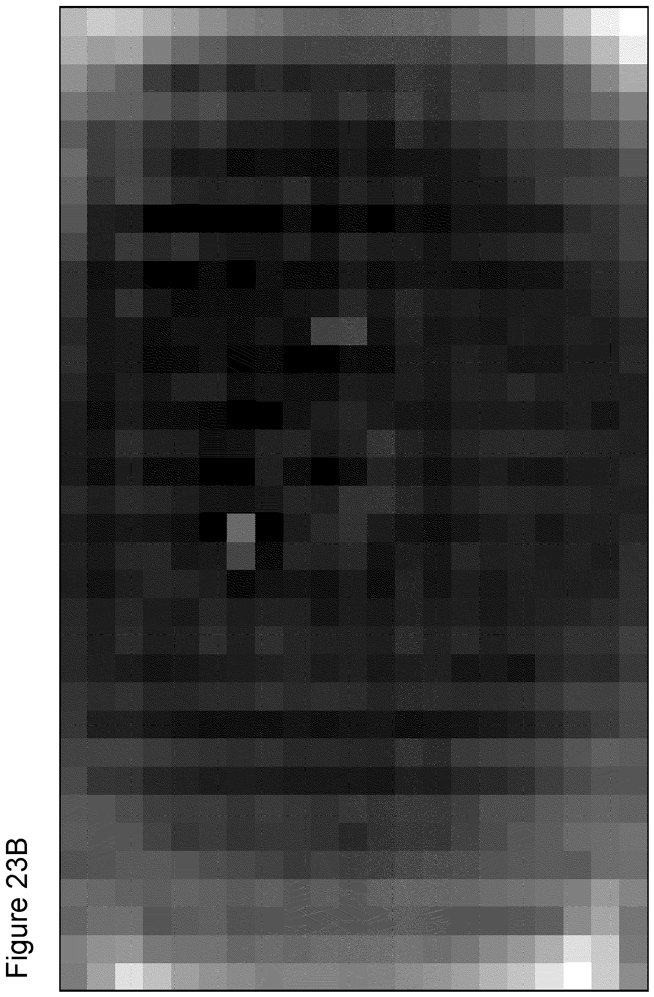

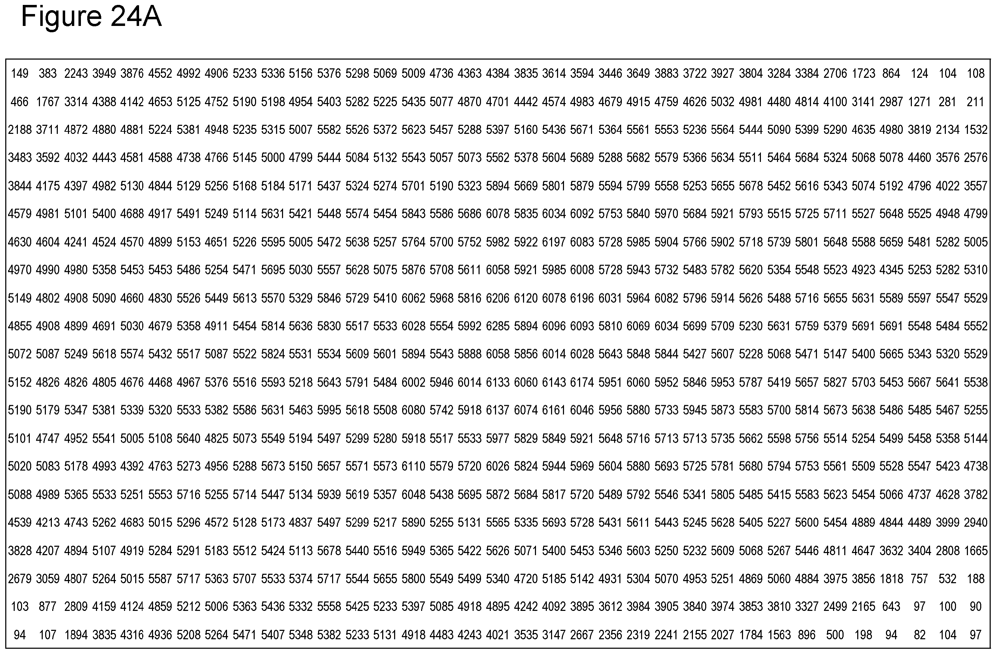

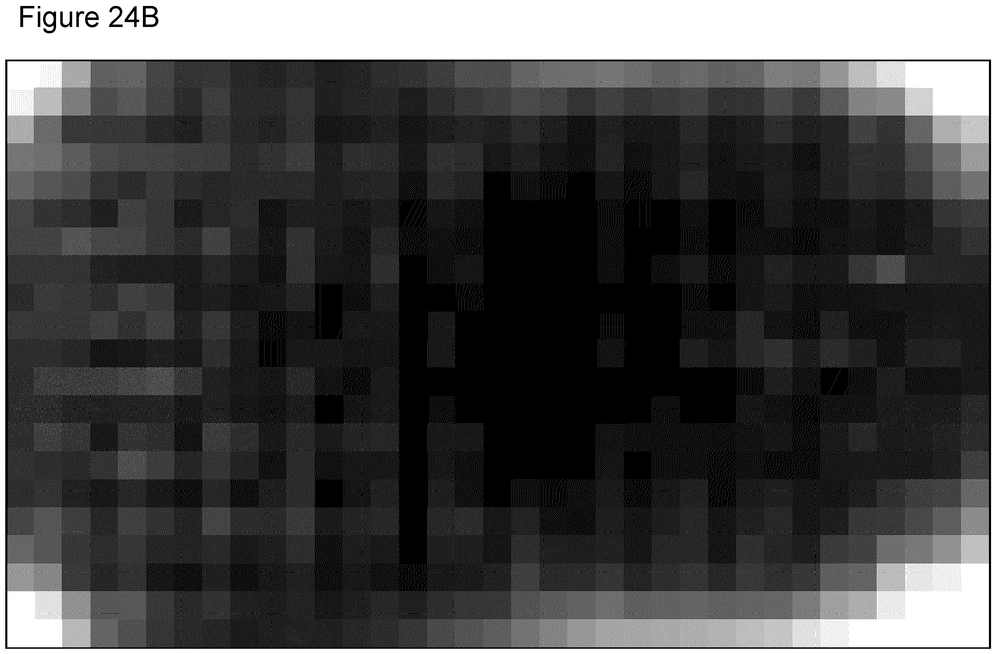

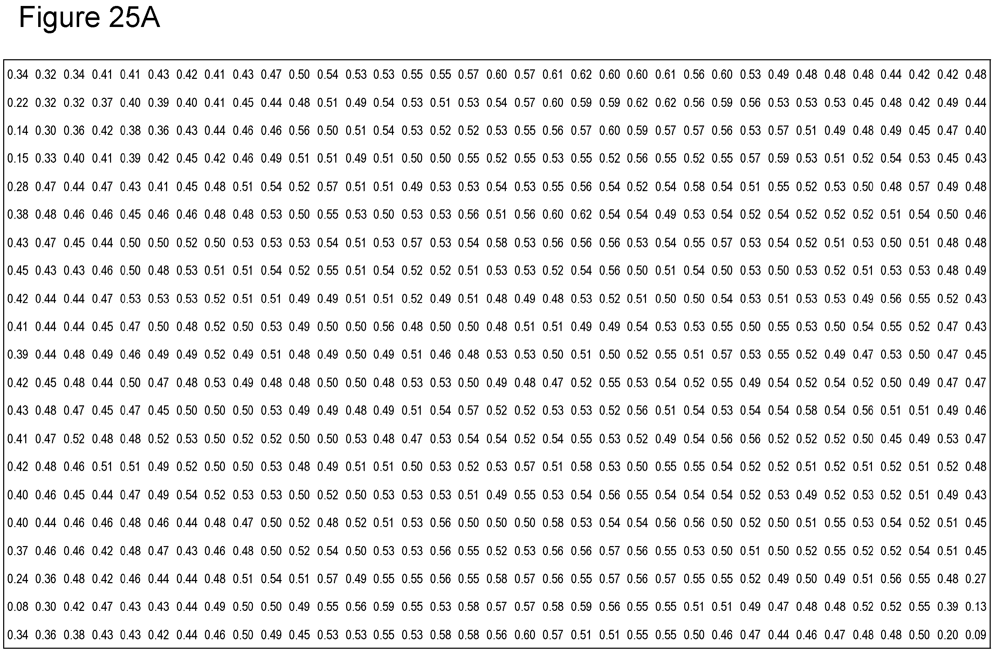

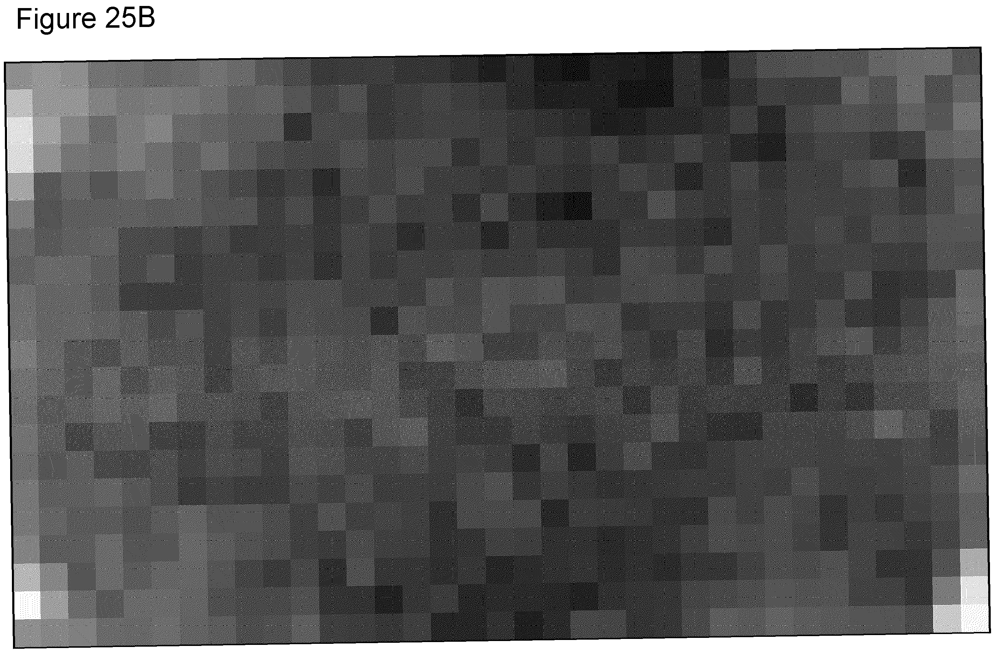

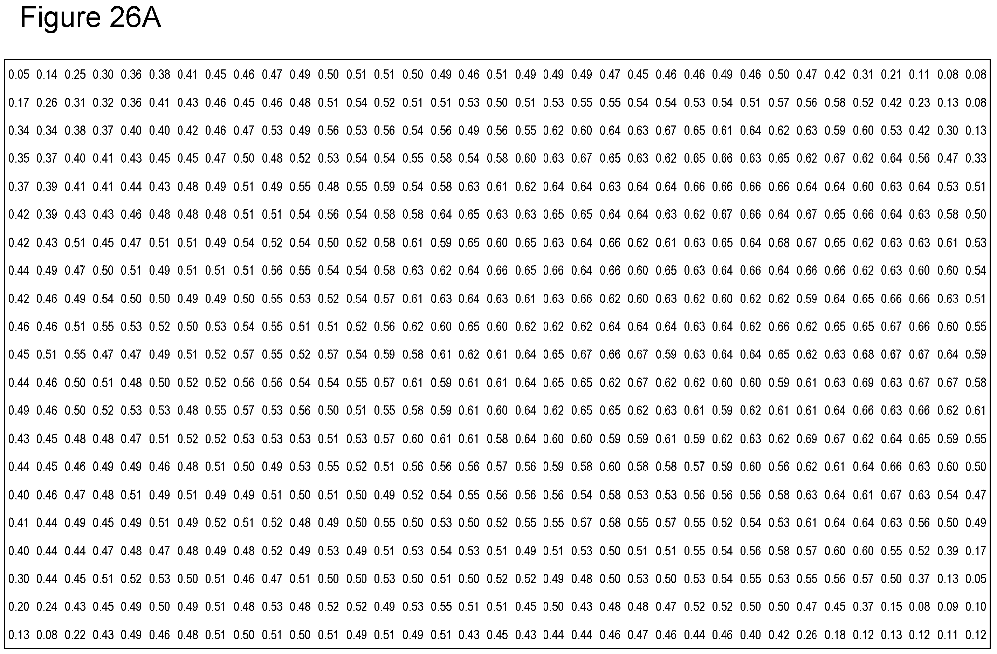

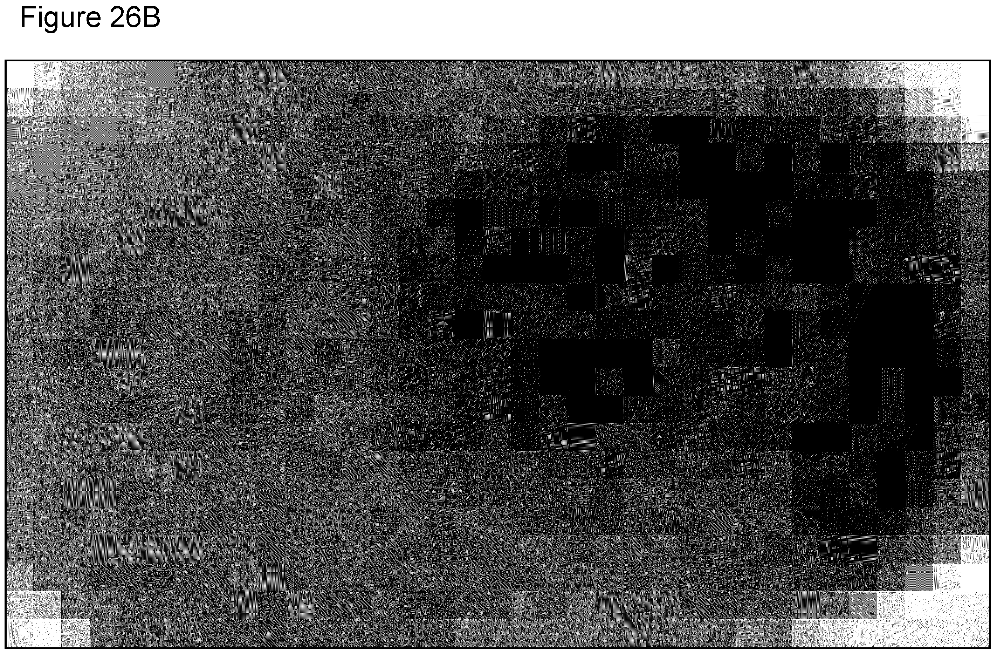

The results of evaluation of the seeding uniformity and evaluation of the homogeneity after induction of differentiation are shown in Figures 23A to 26B. Figures 23A and 23B show the cell seeding density under the single point dispensing condition, Figures 24A and 24B show the cell seeding density under the double point dispensing condition, Figures 25A and 25B show MyHC expression level per cell after induction of differentiation under the single point dispensing condition, and Figures 26A and 26B show MyHC expression level per cell after induction of differentiation under the double point dispensing condition.

**[Table 6]**

| Volume of culture medium in culture vessel before dispensing | 3 mL | 8 mL |
|---|---|---|
| Dispensing method | Single point | Double point |
| Dispensing speed | 890 µL/sec | 890 µL/sec |
| Dispensing volume / total number of dispensing operations | 5 mL/3 times | 5 mL/2 times |
| Shaking | 0 times | 0 times |
| C.V. value at seeding | 20% | 25% |
| Max/Mean value at seeding | 127% | 126% |
| C.V. value after inducing differentiation | 13% | 21% |
| Max/Mean value after inducing differentiation | 125% | 132% |

Also regarding the seeding of C2C12 myoblasts, it was revealed it was possible to seed the cells uniformly by the single point dispensing. Further, also under conditions with higher seeding cell densities as compared to the conditions of Examples 1 to 6, it was revealed that it was possible to seed the cells uniformly (see Table 6 and Figures 23 and 24). Regarding the homogeneity after induction of differentiation, as in Example 7, darker areas on the heat maps are considered as regions with more advanced skeletal muscle differentiation than in surrounding regions, and lighter areas are considered as regions with less advanced skeletal muscle differentiation than in surrounding regions. Thus, smaller differences in the shades of color on the heat maps can be regarded to indicate greater homogeneity of the differentiated cells. Under the double point dispensing condition, differences in the shades of color on the heat maps are observed when comparing the left and right sides of the long side of the culture vessel. In addition, differences in the shades of color on the heat map are also observed in the four corner positions of the culture vessel, and the C.V. value was 21% (see Figure 26). On the other hand, under the single point dispensing condition, the C.V. value was 13%, indicating again in the present Example that differentiated cells with higher homogeneity can be obtained by using the seeding method which achieves high uniformity (see Figure 25).

### Industrial Applicability

According to the methods of the present invention, the cell density in the culture vessel can be controlled without a local bias, which makes it possible to produce homogeneous cells on a large scale with high reproducibility. Further, also when the cultured cells are induced to differentiate, it is possible to produce homogeneous differentiated cells on a large scale with high reproducibility. The present invention is expected to be useful in fields such as cell culturing, drug discovery research, and the production of clinical cell preparations.

## Claims

1. A method of seeding cells using a dispensing device, the method comprising the steps of:
aspirating a cell suspension using the dispensing device; and
dispensing the cell suspension onto a single fixed point in a culture vessel using the dispensing device.

2. The method according to claim 1, wherein the aspirating step and the dispensing step are each performed once or multiple times.

3. The method according to claim 1, further comprising a step of leaving the culture vessel to sit without shaking after the dispensing step, thereby allowing the cells to adhere to the culture vessel.

4. The method according to claim 1, further comprising a step of stirring the cell suspension prior to the aspirating step.

5. The method according to claim 1, further comprising a step of stirring the cell suspension prior to the aspirating step, wherein the aspirating step and the dispensing step are each performed once or multiple times after the stirring step.

6. The method according to claim 4 or 5, wherein after the stirring step, the aspirating step and the dispensing step are completed before the cells in the cell suspension sediment.

7. The method according to claim 1, wherein the single fixed point in the culture vessel is in a center portion of a bottom surface of the culture vessel.

8. The method according to claim 1, wherein the culture vessel is a flat-bottomed culture vessel.

9. The method according to claim 1, wherein the cells are stem cells.

10. The method according to claim 1, wherein the dispensing device is an automated dispensing device.

11. A method of culturing cells in a culture vessel, the method comprising the steps of:
seeding cells into the culture vessel by the method according to claim 1; and
culturing the cells in the culture vessel.

12. The method according to claim 11, further comprising a step of inducing differentiation of the cells.
